(19) Europäisches Patentamt
European Patent Office
Office européen des brevets

(11) EP 2 626 062 A2

(12) EUROPÄISCHE PATENTANMELDUNG

(43) Veröffentlichungstag:
**14.08.2013 Patentblatt 2013/33**

(21) Anmeldenummer: **12196754.1**

(22) Anmeldetag: **12.12.2012**

(51) Int Cl.:
***A61K 8/31*** *(2006.01)* ***A61Q 5/02*** *(2006.01)*
***A61Q 5/06*** *(2006.01)* ***A61Q 5/12*** *(2006.01)*
***A61K 8/891*** *(2006.01)* ***A61K 8/892*** *(2006.01)*
***A61K 8/92*** *(2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **22.12.2011 DE 102011089650**
**22.12.2011 DE 102011089686**
**22.12.2011 DE 102011089638**

(71) Anmelder: **Henkel AG&Co. KGAA**
**40589 Düsseldorf (DE)**

(72) Erfinder: **Krueger, Marcus**
**25373 Ellerhoop (DE)**

(54) **Kosmetisches Mittel, enthaltend (C8 bis C20)-Alkane, Dimethylpolysiloxan, Dimethiconol und mindestens einen festigenden Wirkstoff**

(57) Kosmetische Mittel zur kosmetischen Behandlung keratinhaltiger Fasern, insbesondere menschlicher Haare, enthaltend in einem kosmetisch akzeptablen Träger

(a) eine Mischung aus
i) mindestens einem ($C_8$ bis $C_{20}$)-Alkan
ii) mindestens einem bei 20°C und 1013 mbar flüssigen Polydimethylsiloxan und
iii) mindestens einem Dimethiconol, und

(b) mindestens einen haarkosmetischen Wirkstoff ausgewählt aus mindestens einem Wirkstoff der Gruppe, die gebildet wird aus festigenden Wirkstoffen, quaternären Ammoniumverbindungen und Tensiden,

EP 2 626 062 A2

**Beschreibung**

Die vorliegende Erfindung betrifft Haarpflegemittel, insbesondere Hilfsmittel für die temporäre Umformung, die Reinigung oder die Konditionierung von keratinischen Fasern, insbesondere menschlichen Haaren.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Während bei der permanenten Umformung die chemische Struktur der keratinhaltigen Faser durch Reduktion und Oxidation modifiziert wird, findet bei der temporären Umformung keine solche Modifikation des chemischen Struktur statt. Entsprechende Mittel zur temporären Verformung enthalten als festigenden Wirkstoff üblicherweise synthetische Polymere und/oder Wachse. Mittel zur Unterstützung der temporären Umformung keratinhaltiger Fasern können beispielsweise als Haarspray, Haarwachs, Haargel, Haarschaum konfektioniert werden.

Die wichtigste Eigenschaft eines Mittels zur temporären Verformung keratinischer Fasern, im Folgenden auch Stylingmittel genannt, besteht darin, den behandelten Fasern in der neu modellierten Form - d.h. einer den Fasern aufgeprägten Form - einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten festigenden Wirkstoffe bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar und mild zu Haar und Haut sein.

Um den unterschiedlichen Anforderungen gerecht zu werden, wurde bereits als festigende Wirkstoffe eine Vielzahl von synthetischen Polymeren entwickelt, die in Stylingmitteln zur Anwendung kommen. Die Polymere lassen sich in kationische, anionische, nichtionische und amphotere festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen. Ähnliche Probleme ergeben sich, wenn Wachse als festigender Wirkstoff im Stylingmittel Einsatz finden.

Kosmetische Haarbehandlungsmittel für die Reinigung und/oder die Pflege der Haare sind lange bekannt und werden regelmäßig verbessert bzw. den wechselnden Bedürfnissen der Verbraucher angepasst. Beispielsweise erwarten Verbraucher von einem modernen Haarreinigungsmittel, dass es die Haare u.a. reinigt, glättet, leicht kämmbar, weich, glänzend sowie leicht frisierbar macht, mit Feuchtigkeit versorgt, gegen Schuppen wirkt und dem Haar mehr Volumen verleiht, damit aus Zeit-, Kosten- und Umweltgesichtspunkten kein zweites Haarbehandlungsmittel angewendet werden muss. Es ist eine Vielzahl von Wirkstoffen bekannt, welche in einem Haarreinigungsmittel eine oder mehrere der zuvor genannten Anforderungen erfüllen können. Die Anwesenheit einer großen Menge unterschiedlicher Wirkstoffe kann ein Haarreinigungsmittel jedoch destabilisieren, weshalb üblicherweise versucht wird, mit einer geringen Anzahl gut verträglicher Wirkstoffe möglichst viele der zuvor genannten Anforderungen zu erfüllen.

Häufig wiederholte Haarbehandlungen, insbesondere reduktive und oxidative Haarbehandlungen, sowie UV-Lichteinwirkung oder Fönhitze können Haare in ihrer Struktur dennoch schädigen.

Eine Folge davon ist, dass sie optisch und haptisch unattraktiv erscheinen.

Ein wichtiges Ziel bei der Herstellung von Haarreinigungs- und/oder Konditioniermitteln ist daher die optischen und haptischen Eigenschaften gesunder Haare zu erhalten bzw. wieder herzustellen.

Es ist bekannt, zur Erhöhung des Haarglanzes, der Verbesserung des Griffs und der Kämmbarkeit von Haaren Silikone in Haarreinigungs- und Konditioniermitteln einzusetzen. Zu diesem Zweck werden in der WO 1997/038667 Haarbehandlungszusammensetzungen vorgeschlagen, die Silikone einer besonders kleinen Partikelgröße in einer wässrigen Basis enthalten.

Aus der DE 19757508 ist weiterhin bekannt, Haarbehandlungsmitteln zur Haarglanzverbesserung eine Substanz mit einem Brechungsindex oberhalb von 1,48 hinzuzufügen.

Es besteht weiterhin der Bedarf nach Haarreinigungs- und/oder Konditioniermitteln, die einen Konditionierungsvorteil für optisch und haptisch unattraktive Haare bieten, ohne dass die Reinigungswirkung- und/oder die Pflegewirkung

gemindert wird.

Aufgabe der vorliegenden Erfindung war es daher, ein Mittel zur temporären Verformung keratinhaltiger Fasern zur Verfügung zu stellen, das sich durch einen hohen Haltegrad bzw. durch eine hohe Pflegewirkung auszeichnet. Der vorliegenden Erfindung lag weiterhin die Aufgabe zugrunde, ausgewogene und pflegende Haarreinigungs- und/oder Haarkonditioniermittel bereitzustellen, die den damit behandelten trockenen Haaren mehr Glanz und eine verbesserte Haarsensorik verleihen. Insbesondere sollten die behandelten Haare ein lang anhaltendes Gefühl der Feuchtigkeit, Geschmeidigkeit und Weichheit aufweisen. Eine Beschwerung der Haare sollte vermieden werden. Ein weiteres Ziel der Erfindung bestand darin pflegende Wirkstoffe bzw. Wirkstoffkombinationen für Haarreinigungs- und/oder Haarkonditioniermittel zu finden, die sich einfach in die jeweiligen Mittel einarbeiten lassen, ohne die Textur und/oder die Schaumeigenschaften der Mittel negativ zu verändern.

Es wurde gefunden, dass nachfolgende kosmetische Mittel die Aufgabe in hohem Maße erfüllen. Ein erster Erfindungsgegenstand sind kosmetische Mittel zur kosmetischen Behandlung keratinhaltiger Fasern, insbesondere menschlicher Haare, enthaltend in einem kosmetisch akzeptablen Träger

(a) eine Mischung aus

i) mindestens einem ($C_8$ bis $C_{20}$)-Alkan
ii) mindestens einem bei 20°C und 1013 mbar flüssigen Polydimethylsiloxan und
iii) mindestens einem Dimethiconol, und

(b) mindestens einen haarkosmetischen Wirkstoff ausgewählt aus mindestens einem Wirkstoff der Gruppe, die gebildet wird aus festigenden Wirkstoffen, quaternären Ammoniumverbindungen und Tensiden.

Die erfindungsgemäßen Mittel enthalten die Inhalts- bzw. Wirkstoffe in einem kosmetisch akzeptablen Träger.

Bevorzugte kosmetisch akzeptable Träger sind wässrige, alkoholische oder wässrigalkoholische Medien mit vorzugsweise mindestens 10 Gew.-% Wasser, berechnet auf das Gesamtgewicht des Mittels.

Besonders bevorzugt enthält der erfindungsgemäße kosmetische Träger Wasser (insbesondere in der Menge, dass das kosmetische Mittel, berechnet auf das Gesamtgewicht des Mittels, mindestens 10 Gew.-%, insbesondere mindestens 20,0 Gew.-%, am bevorzugtesten mindestens 40 Gew.-% Wasser enthält.

Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Weiterhin kann der kosmetische Träger bevorzugt 0,01 bis 50 Gew.-%, bevorzugt 0,05 bis 45 Gew.-% und insbesondere 0,1 bis 40 Gew.-% mindestens eines Alkohols enthalten, der ausgewählt sein kann aus Ethanol, Ethyldiglykol, 1-Propanol, 2-Propanol, Isopropanol, 1,2-Propylenglycol, Glycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Sorbitol, Benzylalkohol, Phenoxyethanol oder Mischungen dieser Alkohole.

Bevorzugt sind die wasserlöslichen Alkohole.

Insbesondere bevorzugt sind Ethanol, Ethyldiglykol, 1-Propanol, 2-Propanol, Isopropanol, 1,2-Propylenglycol, Glycerin, Benzylalkohol und/oder Phenoxyethanol sowie Mischungen dieser Alkohole.

Es ist erfindungsgemäß bevorzugt mindestens ein ($C_1$ bis $C_4$)-Monohydroxyalkan in den erfindungsgemäßen Mitteln insbesondere in einer Menge von 1 bis 50 Gew.-% insbesondere von 5 bis 30 Gew.-% einzusetzen. Dies ist wiederum insbesondere für die Konfektionierung als Pumpschaum oder Aerosolschaum bevorzugt.

Besonders bevorzugte wasserlösliche Lösungsmittel als Cosolvens sind Glycerin und/oder Ethylenglykol und/oder 1,2-Propylenglykol in einer Menge von 0 bis 30 Gew.-% bezogen auf das gesamte Mittel.

Die erfindungsgemäß in dem kosmetischen Mittel als Komponente (a) enthaltene Mischung enthält als zwingenden Bestandteil i) mindestens ein ($C_8$ bis $C_{20}$)-Alkan. Es hat sich als bevorzugt herausgestellt, die besagten Alkane des Bestandteils i) der Mischung aus verzweigten Kohlenwasserstoffen ausgewählt werden, insbesondere aus Isoparaffinen. Unter Isoparaffinen versteht der Fachmann gesättigte, apliphatische Kohlenwasserstoffe, deren Molekül mindestens ein Kohlenstoffatom besitzt, an das mindestens drei Kohlenstoffatome binden, i.e. ein Kohlenwasserstoff mit mindestens einem tertiären oder quartären Kohlenstoffatom.

Bevorzugte Kohlenwasserstoffe des Bestandteils i) der Mischung werden ausgewählt aus ($C_{10}$ bis $C_{20}$)-Alkanen, insbesondere verzweigten ($C_{10}$ bis $C_{20}$)-Alkanen, insbesondere ($C_{10}$ bis $C_{20}$)-Isoparaffinen.

Die Isoparaffine liegen bevorzugt als Gemisch von Isoparaffinen mit unterschiedlicher Anzahl an Kohlenstoffatomen innerhalb der erfindungsgemäß vorgegebenen Anzahl an Kohlenstoffatomen vor und umfasst auch Isomerengemische verzweigter Kohlenwasserstoffe mit gleicher Anzahl an Kohlenstoffatomen. Das Isoparaffin-Gemisch kann erfindungsgemäß eine enge Kohlenstoffverteilnug haben wie z.B. C10-12 Isoparaffin, oder eine breite Kohlenstoffverteilung wie z.B. C10-15 Isoparaffin.

Bevorzugte Isoparaffine haben einen Dampfdruck von nicht größer als 2 Torr bei 20°C.

Bevorzugte kosmetische Mittel enthalten als Bestandteil i) der Mischung mindestens ein ($C_8$ bis $C_{20}$)-Alkane ausgewählt aus mindestens einer Verbindung aus der Gruppe von Verbindungen mit der jeweiligen INCI-Bezeichnung C8-9 Isoparaffin, C8-10 Isoparaffin, C10-11 Isoparaffin, C10-13 Isoparaffin, C10-20 Isoparaffin, C11-12 Isoparaffin, C11-13 Isoparaffin, C11-15 Isoparaffin, C13-14 Isoparaffin, C13-16 Isoparaffin, Isodecane, Isoundecane, Isododecane, Isotetradecane, Isohexadecane. Die INCI-Bezeichnung ist eine von der für kosmetische Rohstoffe festgelegte Nomenklatur.

Die in den erfindungsgemäßen Mitteln als Komponente (a) enthaltene Mischung enthält die ($C_8$ bis $C_{20}$)-Alkane, insbesondere die ($C_1$ bis $C_6$)-Isoparaffine, in einer Menge von 25,0 bis 90,0 Gew.-%, insbesondere von 25,0 bis 50,0 Gew.-%, jeweils bezogen auf das Gesamtgewicht der Mischung (a).

Das erfindungsgemäße Mittel enthält in der Mischung der Komponente (a) zwingend mindestens ein bei 20°C und 1013 mbar flüssiges Polydimethylsiloxan.

Diese Polydimethylsiloxane sind bevorzugt dadurch gekennzeichnet, dass sie ausgewählt werden aus mindestens einer Verbindung der Formel (Si-1),

$$(CH_3)_3Si\text{-}[O\text{-}Si(CH_3)_2]_x\text{-}O\text{-}Si(CH_3)_3 \qquad \text{(Si-1)},$$

in der x für eine Zahl von 0 bis 2000, vorzugsweise von 1 bis 500, weiter bevorzugt von 2 bis 200, steht.

Ein erfindungsgemäß bevorzugt geeignetes kosmetisches Mittel ist dadurch gekennzeichnet, dass das bei 20°C und 1013 mbar flüssige Polydimethylsiloxan eine Viskosität von 1,5 bis 20000 cst, insbesondere von 1,5 bis 5000 cSt, jeweils gemessen bei 25 °C mit Hilfe eines Glaskapillarviskosimeters nach der Dow Corning Corporate Testmethode CTM 0004 vom 20. Juli 1970.

Die in den erfindungsgemäßen Mitteln als Komponente (a) enthaltene Mischung enthält die bei 20°C und 1013 mbar flüssige Polydimethylsiloxan, insbesondere das mit der bevorzugten Viskosität, in einer Menge von 1,0 bis 30,0 Gew.-%, insbesondere von 1,0 bis 25,0 Gew.-%, jeweils bezogen auf das Gesamtgewicht der Mischung (a).

Als dritten zwingenden erfindungsgemäßen Bestandteil enthält die Mischung der Komponente (a) des erfindungsgemäßen Mittels mindestens ein Dimethiconol. Vorzugsweise werden die Dimethiconole der Mischung ausgewählt aus mindestens einer Verbindung der Formeln (I) und/oder (II)

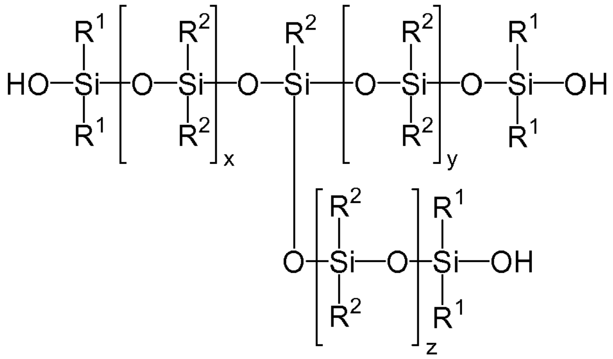
(II)

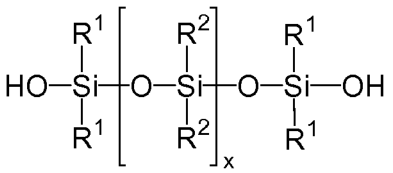
(I)

wobei in den Formeln (I) und (II)

- $R^1$ für Wasserstoff, einen Methylrest, einen $C_2$ bis $C_{30}$ linearen, gesättigten oder ungesättigten Kohlenwasserstoffrest, einen Phenylrest und/oder einen Arylrest,
- $R^2$ für Wasserstoff, einen Methylrest, einen $C_2$ bis $C_{30}$ linearen, gesättigten oder ungesättigten Kohlenwasserstoffrest, einen Phenylrest und/oder einen Arylrest, und
- x, y und z jeweils unabhängig voneinander für eine ganze Zahl von 0 bis 50.000 stehen.

Die erfindungsgemäß bevorzugten Dimethiconole des Bestandteils iii) der Mischung der Komponente (a) des erfindungsgemäßen Mittels sind hochviskos. Erfindungsgemäß besonders bevorzugte kosmetische Mittel sind dadurch gekennzeichnet, dass das besagte Dimethiconol eine Viskosität zwischen 1.000 und 5.000.000 cPs, bevorzugte zwischen 10.000 und 3.000.000 cPs, besonders bevorzugt zwischen 50.000 und 2.000.000 cPs, jeweils gemessen bei 25 °C mit Hilfe eines Glaskapillarviskosimeters nach der Dow Corning Corporate Testmethode CTM 0004 vom 20. Juli 1970.

Die in den erfindungsgemäßen Mitteln als Komponente (a) enthaltene Mischung enthält Dimethiconol, insbesondere die vorgenannten bevorzugten Dimethiconole, in einer Menge von 20,0 bis 30,0 Gew.-%, insbesondere von 22,0 bis 28,0 Gew.-%, jeweils bezogen auf das Gesamtgewicht der Mischung (a).

Die erfindungsgemäße Mischung der Komponente (a) des erfindungsgemäßen Mittels ist bevorzugt jeweils

bezogen auf das Gesamtgewicht des Mittels in einer Menge von 0,01 bis 15,0 Gew.-%, insbesondere von 0,1 bis 10,0 Gew.-%, enthalten.

Die erfindungsgemäßen Mittel enthalten als Komponente (b) bevorzugt mindestens einen festigenden Wirkstoff. Diese Mittel zeichnen sich insbesondere durch einen hohen Haltegrad bzw. durch eine hohe Pflegewirkung aus. Eine Beschwerung der Haare wird vermieden werden. Ein weiteres Ziel der Erfindung bestand darin pflegende Wirkstoffe bzw. Wirkstoffkombinationen für Haarreinigungs- und/oder Haarkonditioniermittel zu finden, die sich einfach in die jeweiligen Mittel einarbeiten lassen, ohne die Textur und/oder die Schaumeigenschaften der Mittel negativ zu verändern.

Dieser festigende Wirkstoff ist von den Bestandteilen i), ii) und iii) der Mischung (a) verschieden. Im Sinne der Erfindung trägt der festigende Wirkstoff im Rahmen der temporären Umformung keratinhaltiger Fasern zum Halt der aufgeprägten Form der Fasern (bei Haaren insbesondere der Halt einer Frisur oder des Haarvolumens) bei. Als eine Testmethode für die festigende Wirkung eines Wirkstoffs wird häufig der so genannte curl-retention - Test angewendet.

Bevorzugte erfindungsgemäße Mittel enthalten die festigenden Wirkstoffe in einer Menge von 0,1 Gew.-% bis 25,0 Gew.-%, besonders bevorzugt von 1,0 Gew.-% bis 20,0 Gew.-%, ganz besonders bevorzugt von 1,0 Gew.-% bis 10,0 Gew.-%, jeweils bezogen auf das Gewicht des Mittels.

Der festigende Wirkstoff des erfindungsgemäßen Mittels wird bevorzugt ausgewählt aus mindestens einem festigenden Polymer und/oder aus mindestens einem Wachs.

Im Rahmen einer allgemeinen Ausführungsform der Erfindung ist es bevorzugt, dass der festigende Wirkstoff ausgewählt wird aus mindestens einem festigenden Polymer aus der Gruppe, die gebildet wird aus festigenden nichtionischen Polymeren, festigenden anionischen Polymeren, festigenden amphoteren Polymeren und festigenden kationischen Polymeren.

Zusätzlich kann das erfindungsgemäße Mittel bevorzugt mindestens ein festigendes kationisches Polymer enthalten.

Die zusätzlichen festigenden kationischen Polymere weisen mindestens eine Struktureinheit auf, die mindestens ein permanent kationisiertes Stichstoffatom enthält. Unter permanent kationisierten Stickstoffatomen sind solche Stickstoffatome zu verstehen, die eine positive Ladung tragen und dadurch eine quartäre Ammoniumverbindung bilden. Quartäre Ammonium-Verbindungen werden meist durch Umsetzung tertiärer Amine mit Alkylierungsmitteln, wie z.B. Methylchlorid, Benzylchlorid, Dimethylsulfat, Dodecylbromid, aber auch Ethylenoxid hergestellt. In Abhängigkeit von dem eingesetzten tertiären Amin sind insbesondere folgende Gruppen bekannt: Alkylammonium-Verbindungen, Alkenylammonium-Verbindungen, Imidazolinium-Verbindungen und Pyridinium-Verbindungen.

Im Sinne dieser Ausführungsform bevorzugte Mittel enthalten die filmbildenden kationischen und/oder festigenden kationischen Polymere in einer Menge von 0,1 Gew.-% bis 20,0 Gew.-%, besonders bevorzugt von 0,2 Gew.-% bis 10,0 Gew.-%, ganz besonders bevorzugt von 0,5 Gew.-% bis 5,0 Gew.-%, jeweils bezogen auf das Gewicht des Mittels.

Die kationischen festigenden Polymere können erfindungsgemäß aus kationischen, quaternisierten Cellulose-Derivaten ausgewählt werden.

Es erweisen sich generell solche kationischen, quaternisierten Cellulosen als im Sinne der Ausführungsform vorteilhaft, die in einer Seitenkette mehr als eine permanente kationische Ladung tragen.

Darunter sind unter den kationischen Cellulosederivaten solche hervorzuheben, die aus Reaktion von Hydroxyethylcellulose mit einem Dimethyldiallylammonium-Reaktanden (insbesondere Dimethyldiallylammoniumchlorid) gegebenenfalls in Gegenwart weiterer Reaktanden hergestellt werden. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat® H 100, Celquat® L 200 von der Firma National Starch vertrieben werden.

Weiterhin eignen sich solche kationischen festigenden Polymere, die mindestens eine Struktureinheit der Formel (I) und mindestens eine Struktureinheit der Formel (VI) und gegebenenfalls mindestens eine Struktureinheit der Formel (V) umfassen

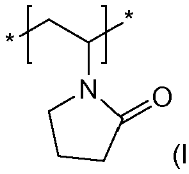
(I)

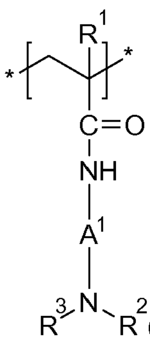
(V)

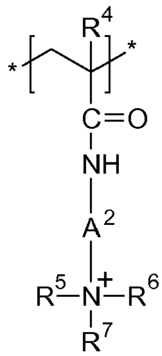
(VI)

worin
$R^1$ und $R^4$ stehen unabhängig voneinander für ein Wasserstoffatom oder eine Methylgruppe,
$A^1$ und $A^2$ stehen unabhängig voneinander für eine Gruppe Ethan-1,2-diyl, Propan-1,3-diyl oder Butan-1,4-diyl,
$R^2$, $R^3$, $R^5$ und $R^6$ stehen unabhängig voneinander für eine ($C_1$ bis $C_4$)-Alkylgruppe,
$R^7$ steht für eine ($C_8$ bis $C_{30}$)-Alkylgruppe.

Geeignete Verbindungen sind beispielsweise als

- Copolymere aus mit Diethylsulfat quaterniertem Dimethylaminoethylmethacrylat, mit N-Vinylpyrrolidon mit der INCI-Bezeichnung Polyquaternium-11 unter den Bezeichnungen Gafquat® 440, Gafquat®734, Gafquat®755 (jeweils Firma ISP) sowie Luviquat PQ 11 PN (Firma BASF SE),
- Copolymere aus Methacryloylaminopropyllauryldimethylammonium chlorid mit N-Vinylpyrrolidon und Dimethylaminopropylmethacrylamid mit der INCI-Bezeichnung Polyquaternium-55 unter den Handelsnamen Styieze® W-10, Styleze® W-20 (Firma ISP),
- Copolymere aus Methacryloylaminopropyllauryldimethylammonium chlorid mit N-Vinylpyrrolidon, N-Vinylcaprolactam und Dimethylaminopropylmethacrylamid mit der INCI-Bezeichnung Polyquaternium-69 unter den Handelsnamen Aquastyle® 300 (Firma ISP),

  im Handel erhältlich.

Als im Sinne der Ausführungsform besonders bevorzugt verwendbare filmbildende und/oder festigende Polymere ausgewählt aus kationischen Polymeren die mindestens eine Struktureinheit enthalten, die ein permanent kationisiertes Stickstoffatom aufweisen, dienen weiterhin solche kationischen filmbildenden und/oder kationischen festigenden Copolymere, die mindestens ein Strukturelement der Formel (M1) enthalten

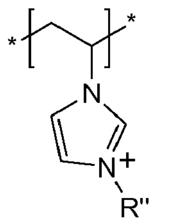
(M1)

worin
R für eine ($C_1$ bis $C_4$)-Alkylgruppe, insbesondere eine Methylgruppe, steht,
und zusätzlich mindestens ein weiteres kationisches und/oder nichtionisches Strukturelement aufweisen.

Es ist wiederum im Rahmen dieser Ausführungsform erfindungsgemäß bevorzugt, wenn in dem erfindungsgemäßen als kationisches filmbildendes und/oder kationisches festigendes Polymer mindestens ein Copolymer (c1) enthalten ist, das neben mindestens einem Strukturelement der Formel (M1) zusätzlich ein Strukturelement der Formel (I) umfasst

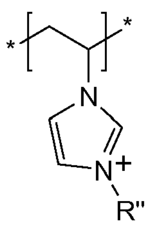
(M1)

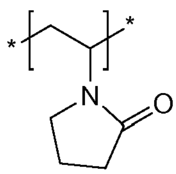
(I).

worin
R für eine ($C_1$ bis $C_4$)-Alkylgruppe, insbesondere eine Methylgruppe, steht.

Ganz besonders bevorzugte kationische filmbildende und/oder kationische festigende Polymere als Copolymere (c1) enthalten 10 bis 30 Mol-%, vorzugsweise 15 bis 25 Mol.-% und insbesondere 20 Mol.-% Struktureinheiten gemäß Formel (M1) und 70 bis 90 Mol.-%, vorzugsweise 75 bis 85 Mol.-% und insbesondere 80 Mol.-% Struktureinheiten gemäß Formel (I).

Hierbei ist besonders bevorzugt, wenn die Copolymere (c1) neben Polymereinheiten, die aus dem Einbau der genannten Struktureinheiten gemäß Formel (M1) und (I) in das Copolymer resultieren, maximal 5 Gew.-%, vorzugsweise maximal 1 Gew.-%, Polymereinheiten enthalten, die auf den Einbau anderer Monomere zurückgehen. Vorzugsweise

sind die Copolymere (c1) ausschließlich aus Struktureinheiten der Formel (M1) mit R" = Methyl und (I) aufgebaut und lassen sich durch die allgemeine Formel (Poly1)

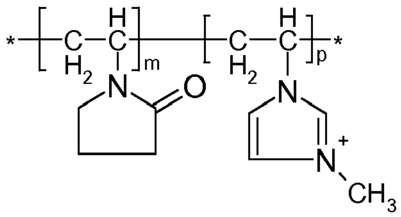

(Poly1)

beschreiben, wobei die Indices m und p je nach Molmasse des Polymers variieren und nicht bedeuten sollen, daß es sich um Blockcopolymere handelt. Vielmehr können Struktureinheiten der Formel (M1) und der Formel (I) im Molekül statistisch verteilt vorliegen.

Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat® Style, Luviquat® FC 370, Luviquat® FC 550, Luviquat® FC 905 und Luviquat® HM 552

Wird in zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat® UltraCare erhältlich.

Zusätzlich zu dem bzw. den Copolymer(en) (c1) oder an dessen bzw. deren Stelle können die erfindungsgemäßen Mittel auch Copolymere (c2) enthalten, die ausgehend vom Copolymer (c1) als zusätzliche Struktureinheiten Struktureinheiten der Formel (VII) aufweisen

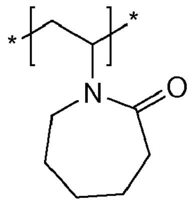

(VII).

Weitere besonders bevorzugte erfindungsgemäße Mittel dieser Ausführungsform sind somit dadurch gekennzeichnet, daß sie als kationisches filmbildendes und/oder kationisches festigendes Polymer mindestens ein Copolymer (c2) enthalten, das mindestens eine Struktureinheit gemäß Formel (M1-a) und mindestens Struktureinheit gemäß Formel (I) und mindestens Struktureinheit gemäß Formel (VII) enthält

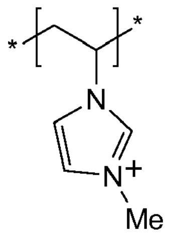

(M1-a)

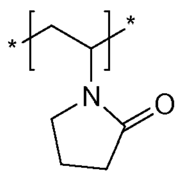

(I)

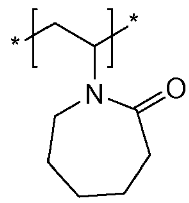

(VII).

Auch hierbei ist es im Rahmen dieser Ausführungsform besonders bevorzugt, wenn die Copolymere (c2) neben Polymereinheiten, die aus dem Einbau der genannten Struktureinheiten gemäß Formel (M1-a), (I) und (VII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c2) ausschließlich aus Struktureinheiten der Formeln (M1-a), (I) und (VII) aufgebaut und lassen sich durch die allgemeine Formel (Poly2)

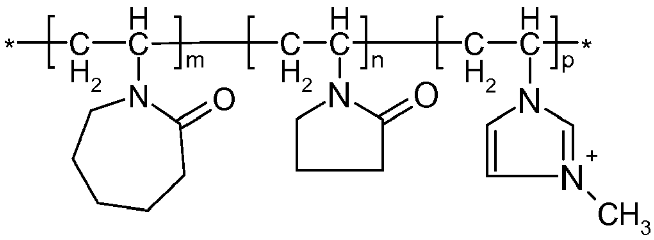 (Poly2)

beschreiben, wobei die Indices m, n und p je nach Molmasse des Polymers variieren und nicht bedeuten sollen, daß es sich um Blockcopolymere handelt. Vielmehr können Struktureinheiten der besagten Formeln im Molekül statistisch verteilt vorliegen.

Wird in zur Kompensation der positiven Ladung des Polymer der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat® Hold erhältlich.

Ganz besonders bevorzugte Copolymere (c2) enthalten 1 bis 20 Mol-%, vorzugsweise 5 bis 15 Mol.-% und insbesondere 10 Mol.-% Struktureinheiten gemäß Formel (M1-a) und 30 bis 50 Mol.-%, vorzugsweise 35 bis 45 Mol.-% und insbesondere 40 Mol.-% Struktureinheiten gemäß Formel (1) und 40 bis 60 Mol.-%, vorzugsweise 45 bis 55 Mol.-% und insbesondere 60 Mol.-% Struktureinheiten gemäß Formel (VII).

Zusätzlich zu dem bzw. den Copolymer(en) (c1) und/oder (c2) oder an dessen bzw. deren Stelle können die erfindungsgemäßen Mittel als filmbildendes kationische und/oder festigendes kationisches Polymer auch Copolymere (c3) enthalten, die als Struktureinheiten Struktureinheiten der Formeln (M1-a) und (I) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße Mittel dieser Ausführungsform sind dadurch gekennzeichnet, daß sie als zusätzliches kationisches filmbildendes und/oder kationisches festigendes Polymer mindestens ein Copolymer (c3) enthalten, das mindestens eine Struktureinheit gemäß Formel (M1-a) und mindestens weitere Struktureinheit gemäß Formel (I) und mindestens weitere Struktureinheit gemäß Formel (VIII) enthält und mindestens weitere Struktureinheit gemäß Formel (IX) enthält

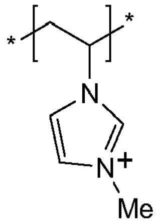 (M1-a) 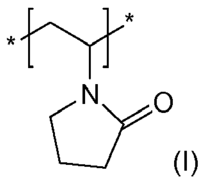 (I) 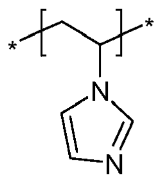 (VIII) 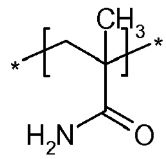 (IX).

Auch hierbei ist es im Rahmen dieser Ausführungsform besonders bevorzugt, wenn die Copolymere (c3) neben Polymereinheiten, die aus dem Einbau der genannten Struktureinheiten gemäß Formel (M1-a), (I), (VIII) und (IX) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c3) ausschließlich aus Struktureinheiten der Formel (M1-a), (I), (VIII) und (IX) aufgebaut und lassen sich durch die allgemeine Formel (Poly3)

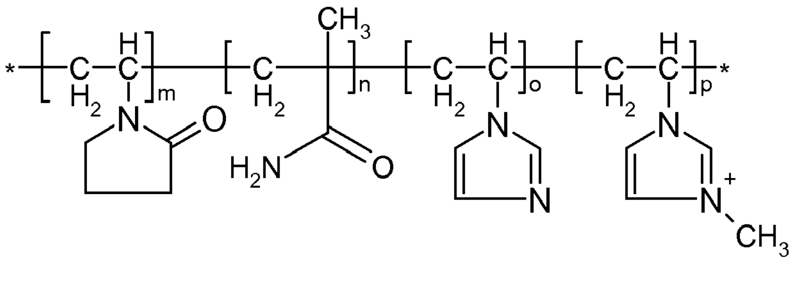 (Poly3)

beschreiben, wobei die Indices m, n, o und p je nach Molmasse des Polymers variieren und nicht bedeuten sollen, daß es sich um Blockcopolymere handelt. Vielmehr können Struktureinheiten der Formeln (M1-a), (I), (VIII) und (IX) im

Molekül statistisch verteilt vorliegen.

Wird in zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat® Supreme erhältlich.

Ganz besonders bevorzugte Copolymere (c3) enthalten 1 bis 12 Mol-%, vorzugsweise 3 bis 9 Mol.-% und insbesondere 6 Mol.-% Struktureinheiten gemäß Formel (M1-a) und 45 bis 65 Mol.-%, vorzugsweise 50 bis 60 Mol.-% und insbesondere 55 Mol.-% Struktureinheiten gemäß Formel (I) und 1 bis 20 Mol.-%, vorzugsweise 5 bis 15 Mol.-% und insbesondere 10 Mol.-% Struktureinheiten gemäß Formel (VIII) und 20 bis 40 Mol.-%, vorzugsweise 25 bis 35 Mol.-% und insbesondere 29 Mol.-% Struktureinheiten gemäß Formel (IX).

Unter den zusätzlichen filmbildenden kationischen und/oder festigenden Polymer ausgewählt aus den kationischen Polymeren mit mindesten einem Strukturelement der obigen Formel (M1), gelten als bevorzugt:

- N-Vinylpyrrolidon/1-Vinyl-3-methyl-1H-imidazoliumchlorid-Copolymere (wie beispielsweise das mit der INCI-Bezeichnung Polyquaternium-16 unter den Handelsbezeichnungen Luviquat® Style, Luviquat® FC 370, Luviquat® FC 550, Luviquat® FC 905 und Luviquat® HM 552 (BASF SE)),
- N-Vinylpyrrolidon/1-Vinyl-3-methyl-1H-imidazoliummethylsulfat-Copolymere (wie beispielsweise das mit der INCI-Bezeichnung Polyquaternium-44 unter den Handelsbezeichnungen Luviquat® Care (BASF SE)),
- N-Vinylpyrrolidon/N-Vinylcaprolactam/1-Vinyl-3-methyl-1H-imidazolium-Terpolymer (wie beispielsweise das mit der INCI-Bezeichnung Polyquaternium-46 unter den Handelsbezeichnungen Luviquat® Care oder Luviquat® Hold (BASF SE)),
- N-Vinylpyrrolidon/Methacrylamid/N-Vinylimidazol/1-Vinyl-3-methyl-1H-imidazoliummethylsulfat-Copolymer (wie beispielsweise das mit der INCI-Bezeichnung Polyquaternium-68 unter der Handelsbezeichnung Luviquat® Supreme (BASF SE)), sowie Gemische aus diesen Polymeren.

Das erfindungsgemäße Mittel kann als zusätzliches festigendes Polymer mindestens ein festigendes nichtionisches Polymer enthalten. Unter einem nichtionischen Polymer wird erfindungsgemäß ein Polymer verstanden, das in einem protischen Lösemittel bei Standardbedingungen im Wesentlichen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Die festigenden nichtionischen Polymere sind in dem erfindungsgemäßen Mittel dieser Ausführungsform bevorzugt in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 10,0 Gew.-%, jeweils bezogen auf das Gewicht des erfindungsgemäßen Mittels, enthalten.

Es sind erfindungsgemäß solche festigende nichtionische Polymere mit mindestens einem Strukturelement der Formel (M2)

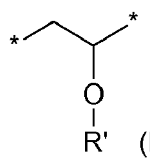
(M2)

bevorzugt geeignet, welche gemäß Formel (M2) als R' ein Wasserstoffatom, eine Acetylgruppe oder eine Propanoylgruppe, insbesondere eine Acetylgruppe, tragen.

Die festigenden nichtionischen Polymere werden wiederum bevorzugt ausgewählt aus mindestens einem Polymer der Gruppe, die gebildet wird, aus

- Homopolymeren und nichtionischen Copolymeren des N-Vinylpyrrolidons,
- nichtionischen Copolymeren des Isobutens.

Geeignete Polyvinylpyrrolidone sind beispielsweise Handelsprodukte wie Luviskol® K 90 oder Luviskol® K 85 der Firma BASF SE. Geeignete Polyvinylalkohole werden beispielsweise unter den Handelsbezeichnungen Elvanol® von Du Pont oder Vinol® 523/540 von der Firma Air Products vertrieben.

Geeignetes Polyvinylacetat wird beispielsweise unter dem Handelsnamen Vinac® als Emulsion von der Firma Air Products vertrieben.

Mittel, die als festigendes nichtionisches Polymer mindestens ein Polymer ausgewählt aus der Gruppe, die gebildet wird aus

- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymere aus N-Vinylpyrrolidon und N-Vinylimidazol und Methacrylamid,
- Copolymere aus N-Vinylpyrrolidon und N-Vinylimidazol und Acrylamid,
- Copolymere aus N-Vinylpyrrolidon mit N,N-Di($C_1$ bis $C_4$)-Alkylamino-($C_2$ bis $C_4$)-alkylacrylamid,
- Copolymere aus N-Vinylpyrrolidon mit N,N-Di($C_1$ bis $C_4$)-Alkylamino-($C_2$ bis $C_4$)-alkylacrylamid, sind erfindungsgemäß ganz besonders bevorzugt.

Weitere mögliche erfindungsgemäße Mittel der Ausführungsform mit zusätzlichem nichtionischen festigendem Polymer sind dadurch gekennzeichnet, daß sie als nichtionisches festigendes Polymer mindestens ein Copolymer enthalten, das mindestens weitere Struktureinheit gemäß Formel (I) und mindestens eine Struktureinheit gemäß Formel (VII) und mindestens eine Struktureinheit gemäß Formel (VIII) enthält

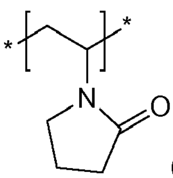 (I)

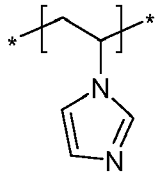 (VII)

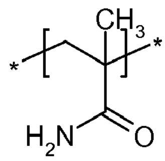 (VIII)

Auch hierbei ist besonders bevorzugt, wenn diese Copolymere neben Polymereinheiten, die aus dem Einbau der genannten Struktureinheiten gemäß Formel (M1-a), (I), (VII) und (VIII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c4) ausschließlich aus Struktureinheiten der Formel (M1-a), (I), (VII) und (VIII) aufgebaut und lassen sich durch die allgemeine Formel (Poly4)

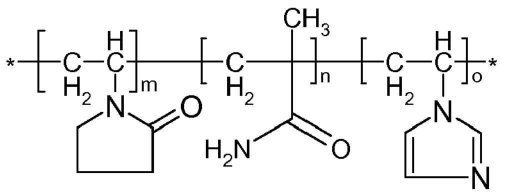 (Poly4)

beschreiben, wobei die Indices m, n, o und p je nach Molmasse des Polymers variieren und nicht bedeuten sollen, daß es sich um Blockcopolymere handelt. Vielmehr können Struktureinheiten der Formeln (I), (VII) und (VIII) im Molekül statistisch verteilt vorliegen.

Ein besonders bevorzugtes Polymer wird dabei ausgewählt aus den Polymeren der INCI-Bezeichnung VP/ Methacrylamide/Vinyl Imidazole Copolymer, die beispielsweise unter dem Handelsnamen Luviset Clear von der Fa. BASF SE erhältlich sind.

Die erfindungsgemäßen Mittel können als festigendes Polymer auch mindestens ein festigendes amphoteres Polymer enthalten. Unter dem Begriff amphotere Polymere werden sowohl solche Polymere, die im Molekül sowohl freie Aminogruppen als auch freie -COOH- oder $SO_3H$-Gruppen enthalten und zur Ausbildung innerer Salze befähigt sind, als auch zwitterionische Polymere, die im Molekül quartäre Ammoniumgruppen und $-COO^-$- oder $-SO_3^-$-Gruppen enthalten, und solche Polymere zusammengefasst, die -COOH- oder $SO_3H$-Gruppen und quartäre Ammoniumgruppen enthalten.

Ein Beispiel für ein erfindungsgemäß einsetzbares festigendes amphoteres Polymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Alkylestern darstellt.

Letztere weisen zusätzlich zu der kationogenen Gruppe bzw. positiv geladenen Gruppe mindestens eine negativ geladene Gruppe im Molekül auf und werden auch als zwitterionische Polymere bezeichnet.

Die festigenden amphoteren Polymere sind in den erfindungsgemäßen Mitteln bevorzugt in Mengen von 0,1

Gew.-% bis 20 Gew.-%, besonders bevorzugt von 0,05 bis 10 Gew.-%, bezogen auf das gesamte Mittel, enthalten. Mengen von 0,1 bis 5 Gew.-% sind ganz besonders bevorzugt.

Weiterhin können als festigende Polymere mindestens ein filmbildendes anionisches Polymer eingesetzt werden.

Bei anionischen Polymeren handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte festigende anionische Polymere sind Acrylsäure-Acrylamid-Copolymere sowie insbesondere Polyacrylamidcopolymere mit Sulfonsäuregruppen-haltigen Monomeren. Ein besonders bevorzugtes festigendes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel®305 der Firma SEPPIC enthalten. Die Verwendung dieses Compounds, das neben der Polymerkomponente eine Kohlenwasserstoffmischung ($C_{13}$-$C_{14}$-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel®600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte festigende anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Weitere bevorzugt einsetzbare festigende anionische Polymere werden ausgewählt aus mindestens einem Polymer der Gruppe, die gebildet wird, aus

- Copolymeren aus Vinylacetat und Crotonsäure (wie sie beispielsweise als Handelsprodukt Aristoflex® A 60 mit der INCI-Bezeichnung VA/Crotonates Copolymer von der Firma CIBA in einer 60 Gew.-%-igen Dispersion in Isopropanol-Wasser vermarktet werden),
- Copolymeren aus Ethylacrylat und Methacrylsäure (wie sie beispielsweise unter dem Handelsnamen Luviflex® Soft mit einer Säurezahl von 84 bis 105 unter der INCI-Bezeichnung Acrylates Copolymer in einer ca. 20 bis 30 Gew.-%igen Dispersion in Wasser von der Firma BASF SE vertrieben werden),
- Polyurethanen mit mindestens einer Carboxylgruppe (wie beispielsweise ein Copolymer aus Isophthalsäure, Adipinsäure, 1,6-Hexandiol, Neopentylglykol und Isophorondiisocyanat wie es unter dem Handelsnamen Luviset PUR mit der INCI-Bezeichung Polyurethane-1 von der Firma BASF SE vertrieben wird).

Als erfindungsgemäßer festigender Wirkstoff finden bevorzugt Wachse ihren Einsatz. Im Rahmen der Erfindung eingesetzte Wachse sind bei 20°C knetbar, fest bis brüchig hart, grob bis feinkristallin, durchscheinend bis opak, jedoch nicht glasartig; über 40 °C ohne Zersetzung schmelzend. Wachse unterscheiden sich von ähnlichen synthetischen oder natürlichen Produkten (z. B. Harzen, plastischen Massen, Metallseifen usw.) darin, dass sie von 40°C bis 90 °C in den schmelzflüssigen, niedrigviskosen Zustand übergehen.

Es sind erfindungsgemäß solche Wachse bevorzugt, die bei 1013 mbar einen Schmelzpunkt im Bereich von 50 °C bis 85 °C, insbesondere von 60°C bis 75 °C, aufweisen.

Bevorzugt werden die Wachse aus pflanzlichen, tierischen und mineralischen Wachsen ausgewählt. Erfindungsgemäß besonders bevorzugte Wachse sind Bienenwachs (Cera Alba), Carnaubawachs, Candelillawachs, Montanwachs, mikrokristalline Wachse (mikrokristalline Paraffine) und Cetylpalmitat. Die erfindungsgemäße Lehre umfasst auch den kombinierten Einsatz von mehreren Wachsen als Komponente (b) des erfindungsgemäßen Mittels.

Die erfindungsgemäßen Zubereitungen enthalten die Wachse vorzugsweise in Mengen von 1,5 bis 60 Gew.-%, bezogen auf die gesamte Zubereitung. Mengen von 5 bis 40 Gew.-%, insbesondere von 10 bis 25 Gew.-%, sind besonders bevorzugt.

Als Komponente (b) enthalten die erfindungsgemäßen Mittel (bezogen auf ihr Gesamtgewicht) bevorzugt 0,1 bis 60 Gew.-% mindestens eines Tensids aus der Gruppe, die gebildet wird aus anionischen, amphoteren/zwitterionischen, nichtionischen und/oder kationischen Tensiden.

Anionische Tenside können den erfindungsgemäßen Mitteln - bezogen auf deren Gesamtgewicht - bevorzugt in Mengen von 0,1 bis 20 Gew.-%, mehr bevorzugt von 0,5 bis 17,5 Gew.-% und insbesondere von 1 bis 15 Gew.-% zugegeben werden.

Zu den geeigneten anionischen Tensiden, die in den erfindungsgemäßen Mitteln eingesetzt werden können, zählen:

- lineare und verzweigte Fettsäuren mit 8 bis 30 C-Atomen (Seifen),
- Ethercarbonsäuren der Formel $R-O-(CH_2-CH_2O)_x-CH_2-COOH$, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfat- und/oder Alkylpolyglykolethersulfatsalze der Formel $R-O(CH_2-CH_2O)_x-OSO_3^- X^+$, in der R eine bevorzugt lineare oder verzweigte, gesättigte oder ungesättigte Alkylgruppe mit 8 bis 30 C-Atomen, x = 0 oder 1 bis 12 und X ein Alkali- oder Ammoniumion ist,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel,

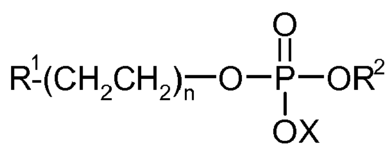

in der $R^1$ bevorzugt für einen aliphatischen Kohlenwasserstoffrest mit 8 bis 30 Kohlenstoff-atomen, $R^2$ für Wasserstoff, einen Rest $(CH_2CH_2O)_nR^1$ oder X, n für Zahlen von 0 bis 10 und X für Wasserstoff, ein Alkali- oder Erdalkalimetall oder $NR^3R^4R^5R^6$, mit $R^3$ bis $R^6$ unabhängig voneinander stehend für einen $C_1$ bis $C_4$ - Kohlenwasserstoffrest, steht.

Bevorzugte anionische Tenside sind Ethercarbonsäuren der zuvor genannten Formel, Acylsarcoside mit 8 bis 24 C-Atomen in der Acylgruppe, Sulfobernsteinsäuremono- und/oder - dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen, Alpha-Olefinsulfonate mit 8 bis 24 C-Atomen und/oder Alkylsulfat- und/oder Alkylpolyglykolethersulfatsalze der zuvor genannten Formel.

Besonders bevorzugte anionische Tenside sind geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten enthalten.

Weiterhin besonders bevorzugte anionische Tenside sind geradkettige oder verzweigte Alkylsulfonate, die einen Alkylrest mit 8 bis 18 und insbesondere mit 10 bis 16 C-Atomen enthalten. Insbesondere bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen.

Amphotere/zwitterionische Tenside können den erfindungsgemäßen Mitteln - bezogen auf deren Gesamtgewicht - bevorzugt in Mengen von 0,1 bis 15 Gew.-%, mehr bevorzugt von 0,5 bis 12,5 Gew.-% und insbesondere von 1 bis 10 Gew.-% zugegeben werden.

Geeignete amphotere/zwitterionische Tenside können ausgewählt sein aus Verbindungen der folgenden Formeln (i) bis (v), in denen der Rest R jeweils für einen geradkettigen oder verzweigten, gesättigten oder ein- bzw. mehrfach ungesättigten Alkyl- oder Alkenlyrest mit 8 bis 24 Kohlenstoffatomen steht,

(i)

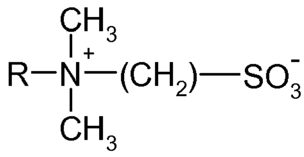

(ii)

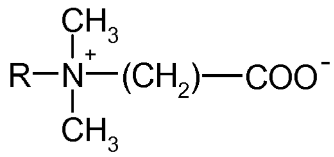

(iii)

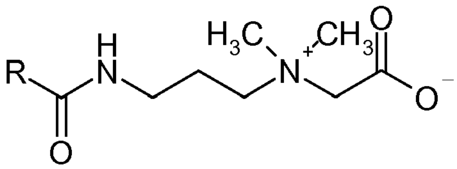

(iv)

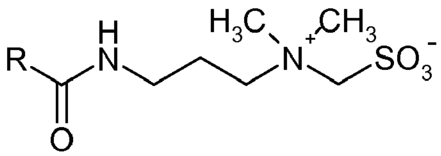

(v)

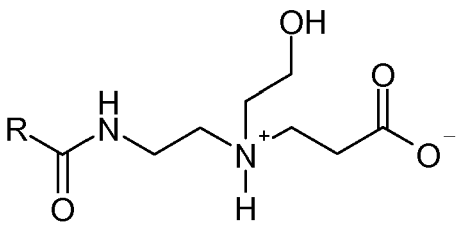

Besonders geeignete amphotere/zwitterionische Tenside sind Alkylamidoalkylbetaine und/oder Alkylampho (di)acetate der zuvor genannten Formeln (i) bis (v).

Zu den insbesondere geeigneten amphoteren/zwitterionischen Tensiden zählen die unter der INCI-Bezeichnung bekannten Tenside Cocamidopropylbetain und Disodium Cocoamphodiacetate.

Kationische Tenside können den erfindungsgemäßen Mitteln - bezogen auf deren Gesamtgewicht - bevorzugt in Mengen von 0,1 bis 10 Gew.-%, bezogen auf das gesamte Mittel, zugegeben werden. Mengen von 0,2 bis 7,5 Gew.-% und insbesondere von 0,3 bis 5 Gew.-% sind besonders bevorzugt.

Geeignete kationische Tenside sind beispielsweise quartäre Ammoniumverbindungen, Esterquats und/oder Amidoamine.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27, Quaternium-83 und Quaternium-87 bekannten Imidazolium-Verbindungen. Die Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart®, Armocare® und Quartamin® vertrieben.

Die Alkylamidoamine werden üblicherweise durch Amidierung natürlicher oder synthetischer Fettsäuren und Fettsäureschnitte mit Dialkylaminoaminen hergestellt. Eine besonders geeignete Verbindung aus dieser Substanzgruppe stellt das unter der Bezeichnung Tegoamid® S 18 im Handel erhältliche Stearamidopropyldimethylamin dar.

Nichtionische Tenside und/oder nichtionische Emulgatoren können den erfindungsgemäßen Mitteln

- bezogen auf deren Gesamtgewicht - bevorzugt in einer Menge von 0,1 bis 15 Gew.-%, bevorzugt 0,5 bis 12,5 Gew.-% und insbesondere 1 bis 10 Gew.-% zugesetzt werden.

Zu den geeigneten nichtionischen Tensiden/Emulgatoren zählen beispielsweise

- $C_8$-$C_{30}$-Fettsäuremono- und -diester von Anlagerungsprodukten von 1 bis 30 Mol Ethylenoxid an Glycerin,
- Aminoxide,
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- Sorbitanfettsäureester und Anlagerungeprodukte von Ethylenoxid an Sorbitanfettsäureester wie beispielsweise Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine und/oder
- Alkylpolyglucoside.

Für den Fall, dass ein nichtionisches Tensid in den Haarbehandlungsmitteln eingesetzt wird, sind Alkyloligoglucoside, insbesondere Alkyloligoglucoside auf der Basis von gehärtetem $C_{12/14}$-Kokosalkohol mit einem DP von 1-3, wie sie beispielsweise unter der INCI-Bezeichnung "Coco-Glucoside" im Handel erhältlich sind, bevorzugt.

Weiterhin bevorzugte nichtionische Tenside sind die $C_8$-$C_{30}$-Fettsäuremono- und -diester von Anlagerungsprodukten von 1 bis 30 Mol Ethylenoxid an Glycerin. Besonders bevorzugt sind die $C_{10}$-$C_{16}$-Fetsäuremono- und -diester von Anlagerungsprodukten von 1 bis 10 Mol Ethylenoxid an Glycerin. Insbesondere bevorzugt ist das unter der INCI-Bezeichnung bekannte PEG-7 Glyceryl Cocoate.

In einer ersten bevorzugten Ausführungsform enthalten erfindungsgemäße Haarreinigungs-und/oder Haarkonditioniermittel - bezogen auf ihr Gesamtgewicht - als Komponente (b)

(a) (i) 0,1 bis 10 Gew.-%, mehr bevorzugt 0,2 bis 7,5 Gew.-% und insbesondere 0,3 bis 5 Gew.-% mindestens eines kationischen Tensids aus der Gruppe der quartären Ammoniumverbindungen, der Esterquats und der Amidoamine und/oder

(ii) 0,1 bis 20 Gew.-%, bevorzugt 0,5 bis 17,5 Gew.-% und insbesondere 1 bis 15 Gew.-% mindestens eines anionischen Tensids aus der Gruppe der Alkyl(ether)sulfate, der Ethercarbonsäuren, der Sulfobernsteinsäuremono- und -dialkylester, der Olefinsulfonate und der Acylsarcoside und/oder

(iii) 0,1 bis 15 Gew.-%, bevorzugt 0,5 bis 12,5 Gew.-% und insbesondere 1 bis 10 Gew.-% mindestens eines amphoteren/zwitterionischen Tensids aus der Gruppe der Alkylbetaine, der Alkylamidoalkylbetaine und der Alkylampho(di)acetate und/oder

(iv) 0,1 bis 15 Gew.-%, bevorzugt 0,5 bis 12,5 Gew.-% und insbesondere 1 bis 10 Gew.-% mindestens eines nichtionischen Tensids aus der Gruppe der Anlagerungsprodukte von Ethylenoxid und/oder Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, der Fettsäuremono- und -diester von Anlagerungsprodukten von Ethylenoxid an Glycerin, der Anlagerungsprodukte von Ethylenoxid an Rizinusöl und gehärtetes Rizinusöl, der Aminoxide, der Anlagerungsprodukte von Ethylenoxid an Fettsäurealkanolamide und Fettamine, und der Alkylpolyglucoside.

Dabei ist es wiederum besonders bevorzugt, wenn die Mischung der Komponente (b) des erfindungsgemäßen Mittels, insbesondere in den bevorzugten Ausführungsformen der Mischung *(vide infra),* jeweils bezogen auf das Gesamtgewicht des Mittels in einer Menge von 0,01 bis 15,0 Gew.-%, insbesondere von 0,1 bis 10,0 Gew.-%, enthalten ist.

Erfindungsgemäß besonders bevorzugt innerhalb dieser Ausführungsform sind Mittel, die neben der Komponente (a) jeweils mindestens ein Tensid aus den zuvor genannten Gruppen (ii) und (iii) enthalten. Zur Herstellung besonders milder und die Kopfhaut schonender Haarreinigungsmittel ist es innerhalb dieser Ausführungsform besonders vorteilhaft, wenn die Tenside aus den Gruppen (ii) und (iii) ein Gewichtsverhältnis von 4 : 1 bis 1 : 3, bevorzugt von 3,5 :

1 bis 1 : 2 und insbesondere von 3 : 1 bis 1 : 1 aufweisen.

Weiterhin besonders bevorzugt innerhalb dieser Ausführungsform sind Haarreinigungsmittel, die neben der Komponente (a) jeweils mindestens ein Tensid aus den zuvor genannten Gruppen (ii), (iii) und (i) enthalten.

Weiterhin besonders bevorzugt innerhalb dieser Ausführungsform sind Haarreinigungsmittel, die neben der Komponente (a) jeweils mindestens ein Tensid aus den zuvor genannten Gruppen (ii), (iii) und (iv) enthalten.

Innerhalb dieser Ausführungsform ist es besonders vorteilhaft, wenn die Tenside aus den Gruppen (ii), (iii) und (iv) ein Gewichtsverhältnis von (3-5) : (1-3) : (1-2) aufweisen.

Weiterhin besonders bevorzugt innerhalb dieser ersten Ausführungsform sind Mittel, die neben der Komponente (a) mindestens ein Tensid aus der zuvor genannten Gruppe (i) enthalten.

Enthalten die erfindungsgemäßen Mittel als Komponente (b) mindestens einen festigenden Wirkstoff, so können diese Mittel bevorzugt zusätzlich mindestens ein nichtionisches Tensid und/oder mindestens ein kationisches Tensid enthalten. Bevorzugte Vertreter dieser Tenside sind die oben genannten Vertreter dieser Klassen.

Im Rahmen einer weiteren bevorzugten Ausführungsform enthält das erfindungsgemäße Mittel als Komponente (b) eine Kombination aus (b1) mindestens einer quaternären Ammoniumverbindung mit (b2) mindestens einen Ölkörper. Diese erfindungsgemäß bevorzugten Haarkonditioniermittel weisen hervorragende Eigenschaften in der Anwendung auf den Haaren auf. Neben der Pflege verleihen sie den damit behandelten Haaren bessere optische und haptische Eigenschaften. Behandelte trockene Haare weisen einen verbesserten Haargriff und erhöhten Haarglanz auf. Sie fühlen sich weich, geschmeidig und elastisch an. Die erfindungsgemäß bevorzugte Wirkstoffkombination dieser Ausführungsform lässt sich sehr gut in eine wässrige oder wässrigalkoholische Basis einarbeiten.

Erfindungsgemäß bevorzugte Haarbehandlungsmittel dieser Ausführungsform enthalten mindestens eine quaternäre Ammoniumverbindung (b1) ausgewählt aus mindestens einer Verbindung der Gruppe, die gebildet wird aus

a1-1) der Esterquats und/oder
a1-2) der quarternären Imidazoline der Formel (Tkat2),

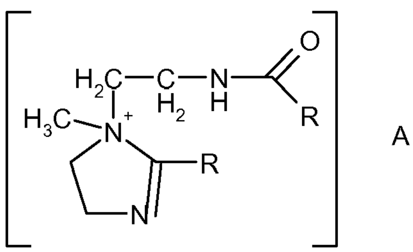

in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
a1-3) der kationische Tenside der Formel (Tkat1-1)

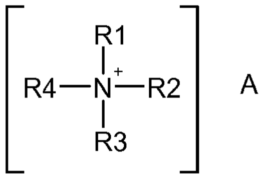

In welcher die Reste R1, R2, R3 und R4 jeweils unabhängig voneinander stehen für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann und A steht für ein physiologisch verträgliches Anion, und/oder
a1-4) der Amine und/oder kationisierten Amine und/oder
a1-5) Poly(methacryloyloxyethyltrimethylammoniumverbindungen) und/oder;
a1-6) quaternisierten Cellulose-Derivaten, insbesondere Polyquaternium 10, Polyquaternium-24, Polyquaternium-27, Polyquaternium-67, Polyquaternium-72, und/oder
a1-7) kationischen Alkylpolyglycoside und/oder
a1-8) kationisiertem Honig und/oder
a1-9) kationischen Guar-Derivaten und/oder
a1-10) Chitosan und/oder

a1-11) polymeren Dimethyldiallylammoniumsalzen und deren Copolymeren mit Estern und Amiden von Acrylsäure und Methacrylsäure, insbesondere Polyquaternium-7 und/oder
a1-12) Copolymeren des Vinylpyrrolidons mit quaternierten Derivaten des Dialkylaminoalkylacrylats und -methacrylats, insbesondere Polyquaternium-11 und/oder
a1-13) Vinylpyrrolidon-Vinylimidazoliummethochlorid-Copolymeren, insbesondere Polyquaternium-16 und/oder
a1-14) quaterniertem Polyvinylalkohol und/oder
a1-15) Polyquaternium-74,
a1-16) Polyquaternium-71,
a1-17) mindestens ein kationisches Keratinhydrolysat der Formel (K1)

R'-X-R" (K1),

in der

- R' für einen geradkettigen oder verzweigten, gesättigten oder ungesättigten Kohlenwasserstoffrest mit 11 bis 24 Kohlenstoffatomen steht,
- R" ein Protein, ein Peptid oder ein Proteinhydrolysat bedeutet,
- X für $-N^+(R^{III}_2)R^{IV}-$ oder $-N(R^{III})R^{IV}-$ oder $-C(O)-N(R^V)R^{VI}-$ steht,
- $R^{III}-(CH_2)_x-CH_3$ mit x = 0 - 22 bedeutet und
- $R^{IV}-CH_2-CH(OH)-CH_2-$ oder $-(CH_2)_x-$ mit x = 0 - 22 bedeutet;
- $R^V$ und $R^{VI}$ unabhängig voneinander für -H oder $-(CH_2)_x-CH_3$ mit x = 0 - 22 stehen; mit der Maßgabe, daß R" für Keratin oder ein Keratinhydrolysat stehen, in einer Gesamtmenge von 0,1 bis 10 Gew.-%, und

Erfindungsgemäß bevorzugte Haarbehandlungsmittel dieser Ausführungsform enthalten die quaternären Ammoniumverbindungen in einer Gesamtmenge von 0,1 bis 10 Gew.-%. Besonders bevorzugte erfindungsgemäße Haarbehandlungsmittel sind dadurch gekennzeichnet, dass sie 0,1 bis 8,0 Gew.-%, vorzugsweise 0,2 bis 8,0 Gew.-%, weiter bevorzugt 0,5 bis 8,0 Gew.-%, noch weiter bevorzugt 0,5 bis 6 Gew.-%, noch weiter bevorzugt 1,0 bis 6 Gew.-% und insbesondere 1,0 bis 5,0 Gew.-% quaternäre Ammoniumverbindungen enthalten.
Quaternäre Ammoniumverbindungen sind prinzipiell monomere kationische oder amphotere Ammoniumverbindungen, monomere Amine, Aminoamide, polymere kationische Ammoniumverbindungen sowie polymere amphotere Ammoniumverbindungen. Aus dieser Vielzahl an möglichen quaternären Ammoniumverbindungen haben sich die folgenden Gruppen als besonders geeignet erwiesen und werden jeweils für sich genommen in einer Menge von 0,1 bis 10,0 Gew.% eingesetzt. Diese Menge wird auch nicht unter- oder überschritten, wenn eine Mischung unterschiedlicher Verbindungen der quaternären Ammoniumverbindungen verwendet wird.
Esterquats gemäß der Formel (Tkat1-2) bilden die erste Gruppe.

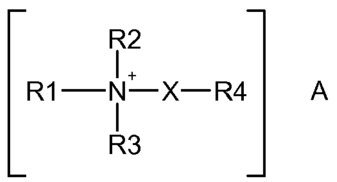

(Tkat1-2)

Hierin sind die Reste R1, R2 und R3 jeweils unabhängig voneinander und können gleich oder verschieden sein. Die Reste R1, R2 und R3 bedeuten:

- ein verzweigter oder unverzweigter Alkylrest mit 1 bis 4 Kohlenstoffatomen, welcher mindestens eine Hydroxylgruppe enthalten kann, oder
- ein gesättigter oder ungesättigter, verzweigter oder unverzweigter oder ein cyclischer gesättigter oder ungesättigter Alkylrest mit 6 bis 30 Kohlenstoffatomen, welcher mindestens eine Hydroxylgruppe enthalten kann, oder
- ein Aryl oder Alkarylrest, beispielsweise Phenyl oder Benzyl,
- den Rest (- X - R4), mit der Maßgabe, daß höchstens 2 der Reste R1, R2 oder R3 für diesen Rest stehen können:

Der Rest -(X - R4) ist mindestens 1 bis 3 mal enthalten.
Hierin steht X für:

1) - (CH2)n- mit n = 1 bis 20, vorzugsweise n = 1 bis 10 und besonders bevorzugt n = 1 - 5, oder

2) -(CH2-CHR5-0)n- mit n = 1 bis 200, vorzugsweise 1 bis 100, besonders bevorzugt 1 bis 50, und besonders bevorzugt 1 bis 20 mit R5 in der Bedeutung von Wasserstoff, Methyl oder Ethyl,

3) eine Hydroxyalkylgruppe mit ein bis vier Kohlenstoffatomen, welche verzweigt oder unverzweigt sein kann, und welche mindestens eine und höchstens 3 Hydroxygruppen enthält. Beispiele sind: $-CH_2OH$, $-CH_2CH_2OH$, -CHOH-CHOH, $-CH_2CHOHCH_3$, $-CH(CH_2OH)_2$, $-COH(CH_2OH)_2$, $-CH_2CHOHCH_2OH$, $-CH_2CH_2CH_2OH$ und Hydroxybutylreste,

und R4 steht für:

1) R6-O-CO-, worin R6 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, oder

2) R7-CO-, worin R7 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann,

und A steht für ein physiologisch verträgliches organisches oder anorganisches Anion und wird an dieser Stelle stellvertretend für alle auch im folgenden beschriebenen Strukturen definiert. Das Anion aller beschriebenen kationischen Verbindungen ist ausgewählt aus den Halogenidionen, Fluorid, Chlorid, Bromid, Iodid, Sulfaten der allgemeinen Formel $RSO_3^-$, worin R die Bedeutung von gesättigtem oder ungesättigtem Alkylresten mit 1 bis 4 Kohlenstoffatomen hat, oder anionischen Reste organischer Säuren wie Maleat, Fumarat, Oxalat, Tartrat, Citrat, Lactat oder Acetat. Solche Produkte werden beispielsweise unter den Warenzeichen Rewoquat®, Stepantex®, Dehyquart®, Armocare® und Akypoquat® vertrieben. Die Produkte Armocare® VGH-70, Dehyquart® F-75, Dehyquart® C-4046, Dehyquart® L80, Dehyquart® F-30, Dehyquart® AU-35, Rewoquat® WE18, Rewoquat® WE38 DPG, Stepantex® VS 90 und Akypoquat® 131 sind Beispiele für diese Esterquats.

Weitere erfindungsgemäß besonders bevorzugte Verbindungen der Formel (Tkat1-2) zählen zur Formel (Tkat1-2.1), den kationischen Betainestern.

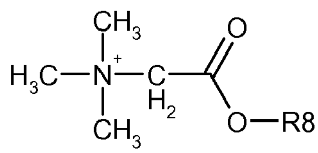

(Tkat1-2.1)

R8 entspricht in seiner Bedeutung R7.

Besonders bevorzugt sind die Esterquats mit den Handelsbezeichnungen Armocare® VGH-70, sowie Dehyquart® F-75, Dehyquart® L80, Stepantex® VS 90 und Akypoquat® 131.

Eine weitere Gruppe sind quartäre Imidazolinverbindungen. Die im folgenden dargestellte Formel (Tkat2) zeigt die Struktur dieser Verbindungen.

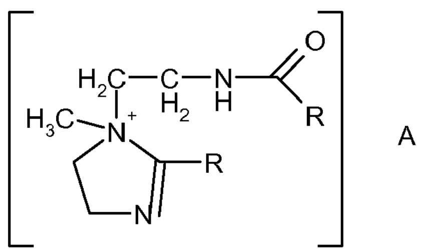

(Tkat2)

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel (Tkat2) enthalten für R jeweils den gleichen Kohlenwasserstoffrest. Die Kettenlänge der Reste R beträgt bevorzugt 12 bis 21 Kohlenstoffatome. A steht für ein Anion wie zuvor beschrieben. Besonders erfindungsgemäße Beispiele sind beispielsweise unter den INCII - Bezeichnungen Quaternium-27, Quaternium-72, Quaternium-83 und Quaternium-91 erhältlich. Höchst bevorzugt ist erfindungsgemäß Quaternium-91.

Kationische Tenside der Formel (Tkat1-1) können ebenfalls verwendet werden.

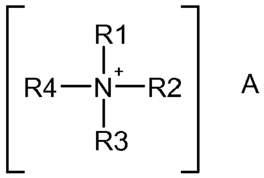

(Tkat1)

In der Formel (Tkat1) stehen R1, R2, R3 und R4 für jeweils unabhängig voneinander für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann. A steht für ein physiologisch verträgliches Anion, beispielsweise Halogenide wie Chlorid oder Bromid sowie Methosulfate.

Beispiele für Verbindungen der Formel (Tkat1) sind Lauryltrimehtylammoniumchlorid, Cetyltrimethylammoniumchlorid, Cetyltrimethylammoniumbromid, Cetyltrimethylammoniummethosulfat, Dicetyldimethylammoniumchlorid, Tricetylmethylammoniumchlorid, Stearyltrimethylammoniumchlorid, Distearyldimethylammoniumchlorid, Lauryldimethylbenzylammoniumchlorid, Behenyltrimethylammoniumchlorid, Behenyltrimethylammoniumbromid, Behenyltrimethylammoniummethosulfat.

In einer besonders bevorzugten Ausführungsform der Erfindung enthalten die erfindungsgemäßen Mittel weiterhin mindestens ein Amin und/oder kationisiertes Amin, insbesondere ein Amidoamin und/oder ein kationisiertes Amidoamin mit den folgenden Strukturformeln:

$$R1 - NH - (CH_2)_n - N^+R^2R^3R^4\ A \qquad (Tkat3)$$

worin R1 ein Acyl-oder Alkylrest mit 6 bis 30 C-Atomen, welche verzweigt oder unverzweigt, gesättigt oder ungesättigt sein können, und wobei der Acylrest und/oder der Alkylrest mindestens eine OH-Gruppe enthalten können, und R2, R3 und R4 jeweils unabhängig voneinander

1) Wasserstoff oder
2) ein Alkylrest mit 1 bis 4 C-Atomen, welcher gleich oder verschieden, gesättigt oder ungesättigt sein kann, und
3) eine verzweigte oder unverzweigte Hydroxyalkylgruppe mit ein bis 4 Kohlenstoffatomen mit mindestens einer und höchstens drei Hydroxygruppen beispielsweise $-CH_2OH$, $-CH_2CH_2OH$, -CHOHCHOH, $-CH_2CHOHCH_3$, $-CH(CH_2OH)_2$, $-COH(CH_2OH)_2$, $-CH_2CHOHCH_2OH$, $-CH_2CH_2CH_2OH$ und Hydroxybutylreste, und

A ein Anion wie zuvor beschrieben und

n eine ganze Zahl zwischen 1 und 10 bedeuten.

Bevorzugt wird eine Zusammensetzung, in welcher das Amin und/oder das quaternisierte Amin gemäß allgemeiner Formeln (Tkat3) ein Amidoamin und/oder ein quaternisiertes Amidoamin ist, worin R1 ein verzweigter oder unverzweigter, gesättigter oder ungesättigter Acylrest mit 6 bis 30 C-Atomen, welcher mindestens eine OH-Gruppe enthalten kann, bedeutet. Bevorzugt ist hierbei ein Fettsäurerest aus Ölen und Wachsen, insbesondere aus natürlichen Ölen und Wachsen. Als Beispiele hierfür kommen Lanolin, Bienen- oder Candellilawachse in Betracht.

Bevorzugt sind auch solche Amidoamine und/oder quaternisierte Amidoamine, in denen R2, R3 und/oder R4 in der Formel (Tkat3) ein Rest gemäß der allgemeinen Formel $CH_2CH_2OR5$ bedeuten, worin R5 die Bedeutung von Alkylresten mit 1 bis 4 Kohlenstoffatomen, Hydroxyethyl oder Wasserstoff haben kann. Die bevorzugte Größe von n in der allgemeinen Formel (Tkat8) ist eine ganze Zahl zwischen 2 und 5.

Die Alkylamidoamine können sowohl als solche vorliegen und durch Protonierung in entsprechend saurer Lösung in eine quaternäre Verbindung in der Zusammensetzung überführt werden. Erfindungsgemäß bevorzugt sind die kationischen Alkylamidoamine.

Beispiele für derartige erfindungsgemäße Handelsprodukte sind Witcamine® 100, Incromine® BB, Mackine® 401und andere Mackine® -Typen, Adogen® S18V, und als permanent kationische Aminoamine: Rewoquat® RTM 50, Empigen® CSC, Swanol® Lanoquat DES-50, Rewoquat® UTM 50, Schercoquat® BAS, Lexquat® AMG-BEO, oder Incroquat® Behenyl HE.

Weitere quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Behenyltrimethylammoniumchlorid, Distearyldimethylammoniumchlorid, Lauryldimethylammoniumchlorid, Lauryldimethylbenzylammoniumchlorid und Tricetylmethylammoniumchlorid. Die langen Alkylketten der oben genannten Tenside weisen bevorzugt 10 bis 22 Kohlenstoffatome auf. Die zuvor genannten kationischen Tenside können einzeln oder in beliebigen Kombinationen miteinander verwendet werden, wobei Mengen zwischen 0,01 bis 10 Gew.%, bevorzugt in Mengen von 0,01 bis 7,5 Gew.% und ganz besonders bevorzugt in Mengen von 0,1 bis 5,0 Gew.% enthalten. Die allerbesten Ergebnisse werden dabei mit Mengen von 0,1 bis 3,0 Gew. % jeweils bezogen auf die Gesamtzusammensetzung des jeweiligen Mittels erhalten.

Weitere quarternäre Ammoniumverbindungen sind kationische und amphotere Polymer.

Die kationischen und/oder amphoteren Polymere können Homo- oder Copolymere oder Polymere auf Basis natürlicher Polymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl-und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Aus der Vielzahl dieser Polymere haben sich als besonders wirkungsvolle Bestandteile des erfindungsgemäßen Wirkstoffkomplexes erwiesen:

Homopolymere der allgemeinen Formel $-\{CH_2[CR^1COO-(CH_2)_mN^+R^2R^3R^4]\}_n X^-$,

in der $R^1$= -H oder $-CH_3$ ist, $R^2$, $R^3$ und $R^4$ unabhängig voneinander ausgewählt sind aus C1-4-Alkyl-, -Alkenyl- oder -Hydroxyalkylgruppen, m = 1, 2, 3 oder 4, n eine natürliche Zahl und

$X^-$ ein physiologisch verträgliches organisches oder anorganisches Anion ist. Im Rahmen dieser Polymere sind diejenigen erfindungsgemäß bevorzugt, für die mindestens eine der folgenden Bedingungen gilt: $R^1$ steht für eine Methylgruppe, $R^2$, $R^3$ und $R^4$ stehen für Methylgruppen, m hat den Wert 2.

Als physiologisch verträgliches Gegenionen $X^-$ kommen beispielsweise Halogenidionen, Sulfationen, Phosphationen, Methosulfationen sowie organische Ionen wie Lactat-, Citrat-, Tartrat-und Acetationen in Betracht. Bevorzugt sind Methosulfate und Halogenidionen, insbesondere Chlorid.

Geeignete kationische Polymere, die von synthetischen Poylmeren abgeleitet sind, sind beispielsweise Copolymere aus 0,1 bis 50 % (bezogen auf die Gesamt-Anzahl an Monomeren im Copolymer) Monomeren der Formel (K2)

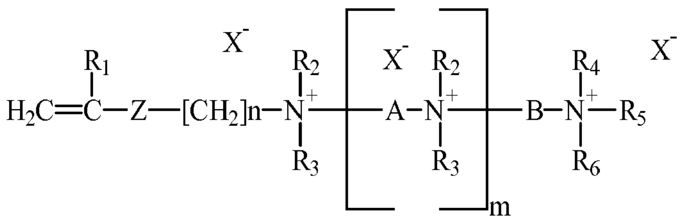

(K2)

in der die weiter oben genannten Definitionen für die Reste $R^1$, $R^2$, $R^3$, $R^4$, $R^5$, $R^6$ sowie die Indices n, m und die Gruppen Z, A, B, X gelten, und Monomeren aus der Gruppe Acrylsäure, Methacrylsäure, alpha-Ethacrylsäure, beta, beta-Dimethylacrylsäure, Methylenmalonsäure, Vinylessigsäure, Allylessigsäure, Ethylidinessigsäure, Propylidinessigsäure, Crotonsäure, Maleinsäure, Fumarsäure, Itaconsäure, Citraconsäure, Mesaconsäure, N-Methacryloylalanin, N-Acryloylhydroxyglycin, Sulfopropylacrylat, Sulfoethylacrylat, Sulfoethylmethacrylat, Sulfoethylmethacrylat, Styrolsulfonsäure, Vinylsulfonsäure, Vinylphosphonsäure, Phosphoethylacrylat, Phosphonoethylacrylat, Phosphopropylacrylat, Phosphonopropylacrylat, Phosphoethylmethacrylat, Phosphonoethylmethacrylat, Phosphopropylmethacrylat und Phosphonopropylmethacrylat sowie die Alkalimetall- und Ammoniumsalze dieser Säuren, sowie optional nichtionischen Monomeren aus der Gruppe Acrylamid, Vinylalkohol, $C_1$-$C_4$-Alkylester von Acrylsäure und/oder von Methacrylsäure, $C_1$-$C_4$-Hydroxyalkylester von Acrylsäure und/oder von Methacrylsäure, insbesondere Ethylenglykol- und Propylenglykolacrylat und -methacrylat, polyalkoxylierte Ester von Acrylsäure und/oder von Methacrylsäure, insbesondere die Polyethylenglykol- und Polypropylenglykolester, Ester von Acrylsäure und/oder von Methacrylsäuremit Polyethylenglykol-

oder Polypropylenglykol-mono($C_1$-$C_{25}$)alkylethern, Vinylacetat, Vinylpyrrolidon und Methylvinylether, wobei die Monomere A2 und A3 gemeinsam 50 bis 99,9 % (bezogen auf die Gesamt-Anzahl an Monomeren im Copolymer) des Copolymers ausmachen.

In der Formel (K2) für das Monomer (A) ist $R^1$ vorzugsweise eine Methylgruppe, und auch $R^2$, $R^3$, $R^4$, $R^5$ und $R^6$ stehen für Methylgruppen. Die Gruppe Z ist vorzugsweise eine -NH-Gruppe, der Index n steht besonders bevorzugt für die Zahl 3.

Je nach Wahl der Gruppen A und B sowie des Index m können verschiedene Monomere der Formel (K2) bevorzugt sein. Ein bevorzugtes Monomer, das den im vorgenannten Absatz genannten Kriterien entspricht, besitzt als Gruppe B darüber hinaus eine $-CH_2-CH(OH)-CH_2-$Gruppe, und der Index m steht für die Zahl 0. Polymere, die solche Monomere enthalten, werden vorzugsweise innerhalb engerer Mengenbereiche eingesetzt. Damit sind erfindungsgemäß bevorzugte Haarbehandlungsmittel dadurch gekennzeichnet, daß sie - bezogen auf ihr Gewicht - 0,001 bis 5 Gew.-%, vorzugsweise 0,0025 bis 2,5 Gew.-%, besonders bevorzugt 0,005 bis 1 Gew.-%, weiter bevorzugt 0,0075 bis 0,75 Gew.-% und insbesondere 0,01 bis 0,5 Gew.-% mindestens eines Copolymers A aus

A1) 0,1 bis 50 %, vorzugsweise 10 bis 50 % (bezogen auf die Gesamt-Anzahl an Monomeren im Copolymer) Monomeren der Formel (K2a)

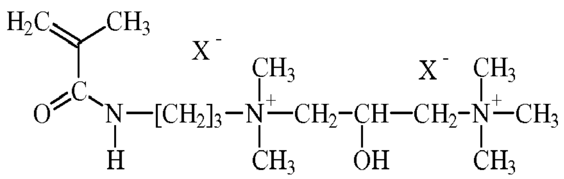

(K2a)

in der

X steht für Chlorid, Sulfat, Methosulfat,

A2) Monomere aus der Gruppe Acrylsäure, Methacrylsäure sowie den Alkalimetall- und Ammoniumsalzen dieser Säuren, wobei das Monomere A2 50 bis 99,9 %, vorzugsweise 50 bis 90 % (bezogen auf die Gesamt-Anzahl an Monomeren im Copolymer) des Copolymers ausmacht;

enthalten.

Ein weiteres bevorzugtes Monomer, das den im vorvorgenannten Absatz genannten Kriterien entspricht, besitzt als Gruppe B darüber hinaus eine $-CH_2-CH(OH)-CH_2-$Gruppe, als Gruppe A eine $-(CH_2)_2-$ oder eine $-(CH_2)_3-$ oder eine $-(CH_2)_4-$Gruppe, und der Index m steht für die Zahl 1. Polymere, die solche Monomere enthalten, werden ebenfalls vorzugsweise innerhalb engerer Mengenbereiche eingesetzt. Damit sind erfindungsgemäß bevorzugte Haarbehandlungsmittel dadurch gekennzeichnet, daß sie - bezogen auf ihr Gewicht - 0,001 bis 5 Gew.-%, vorzugsweise 0,0025 bis 2,5 Gew.-%, besonders bevorzugt 0,005 bis 1 Gew.-%, weiter bevorzugt 0,0075 bis 0,75 Gew.-% und insbesondere 0,01 bis 0,5 Gew.-% mindestens eines Copolymers A aus A1) 0,1 bis 50 %, vorzugsweise 10 bis 50 % (bezogen auf die Gesamt-Anzahl an Monomeren im Copolymer) Monomeren der Formel (K2b)

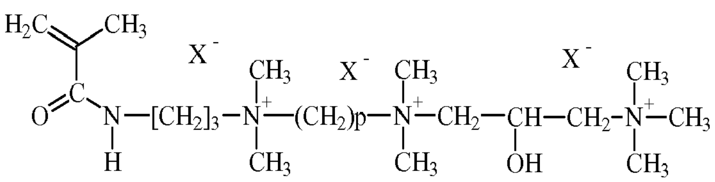

(K2b)

in der

p steht für 2, 3 oder 4,

X steht für Chlorid, Sulfat, Methosulfat,

A2) Monomere aus der Gruppe Acrylsäure, Methacrylsäure sowie den Alkalimetall- und Ammoniumsalzen dieser Säuren, wobei das Monomere A2 50 bis 99,9 %, vorzugsweise 50 bis 90 % (bezogen auf die Gesamt-Anzahl an Monomeren im Copolymer) des Copolymers ausmacht;

enthalten.

Besonders bevorzugte Monomere A2 sind Acrylsäure bzw. ihre Salze (auch gemischt, d.h. teilneutralisierte Acrylsäuren) sowie Acrylamid. Ein bevorzugtes Copolymer A ist ein Copolymer aus dem Monomer (K2a), Natriumacrylat und Acrylamid, wobei folgende Verteilung (in % der insgesamt im Polymer enthaltenen Monomere) bevorzugt ist:

Monomer (K2a):0,1 bis 50 %, vorzugsweise 10 bis 50 %

Natriumacrylat: 10 bis 95 %, vorzugsweise für 50 bis 70 %
Acrylamid: 0 bis 50 %, vorzugsweise 0 bis 30 %
Vorzugsweise enthält ein bevorzugtes Copolymer A folgende Anzahl an den jeweiligen Monomeren:

Monomer (K2a):Werte von 1 bis 12500, vorzugsweise von 2 bis 8000, besonders bevorzugt von 3 bis 4000 und insbesondere von 5 bis 2000
Natriumacrylat: Werte von 1 bis 24000, vorzugsweise von 5 bis 15000, besonders bevorzugt von 10 bis 10000 und insbesondere von 100 bis 4800
Acrylamid: Werte 0, 1, 2, 3, 4, 5, wobei der Wert 0 bevorzugt ist

Zusammenfassend sind erfindungsgemäße Haarbehandlungsmittel bevorzugt, die - bezogen auf ihr Gewicht - 0,001 bis 5 Gew.-%, vorzugsweise 0,0025 bis 2,5 Gew.-%, besonders bevorzugt 0,005 bis 1 Gew.-%, weiter bevorzugt 0,0075 bis 0,75 Gew.-% und insbesondere 0,01 bis 0,5 Gew.-% mindestens eines Copolymers A der allgemeinen Formel (K2c)

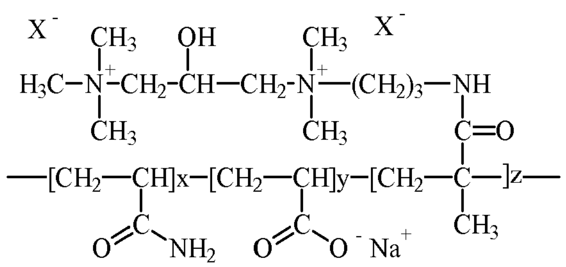

(K2c)

enthalten, in der gilt:

$$x + y + z = Q$$

- Q steht für Werte von 3 bis 55000, vorzugsweise von 10 bis 25000, besonders bevorzugt von 50 bis 15000, weiter bevorzugt von 100 bis 10000, noch weiter bevorzugt von 500 bis 8000 und insbesondere von 1000 bis 5000,
- x steht für (0 bis 0,5) Q, vorzugsweise für (0 bis 0,3) Q und insbesondere für die Werte 0, 1, 2, 3, 4, 5, wobei der Wert 0 bevorzugt ist,
- y steht für (0,1 bis 0,95) Q, vorzugsweise für (0,5 bis 0,7) Q und insbesondere für Werte von 1 bis 24000, vorzugsweise von 5 bis 15000, besonders bevorzugt von 10 bis 10000 und insbesondere von 100 bis 4800,
- z steht für (0,001 bis 0,5) Q, vorzugsweise für (0,1 bis 0,5) Q und insbesondere für Werte von 1 bis 12500, vorzugsweise von 2 bis 8000, besonders bevorzugt von 3 bis 4000 und insbesondere von 5 bis 2000.

Zusätzlich zu dem vorstehend genannten Copolymer oder an seiner Stelle können die erfindungsgemäßen Mittel auch ein Copolymer enthalten, das aus Monomeren der Formel (K2a), Malein- bzw. Fumarsäure (bzw. deren Dinatriumsalzen) und Acrylamid aufgebaut ist. Hierbei ist folgende Verteilung (in % der insgesamt im Polymer enthaltenen Monomere) bevorzugt:

| | |
|---|---|
| Monomer (K2a): | 0,1 bis 50 %, vorzugsweise 10 bis 50 % |
| Maleinsäure bzw. Fumarsäure (bzw. deren Dinatriumsalz): | 10 bis 95%, vorzugsweise für 50 bis 70% |
| Acrylamid: | 0 bis 50 %, vorzugsweise 0 bis 30 % |

Vorzugsweise enthält ein bevorzugtes Copolymer A folgende Anzahl an den jeweiligen Monomeren:

| | |
|---|---|
| Monomer (K2a): | Werte von 1 bis 12500, vorzugsweise von 2 bis 8000, besonders bevorzugt von 3 bis 4000 und insbesondere von 5 bis 2000 |

| | |
|---|---|
| Maleinsäure bzw. Fumarsäure (bzw. deren Dinatriumsalz): | Werte von 1 bis 24000, vorzugsweise von 5 bis 15000, besonders bevorzugt von 10 bis 10000 und insbesondere von 100 bis 4800 |
| Acrylamid: | Werte 0, 1, 2, 3, 4, 5, wobei der Wert 0 bevorzugt ist |

Zusammenfassend sind erfindungsgemäße Haarbehandlungsmittel bevorzugt, die - bezogen auf ihr Gewicht - 0,001 bis 5 Gew.-%, vorzugsweise 0,0025 bis 2,5 Gew.-%, besonders bevorzugt 0,005 bis 1 Gew.-%, weiter bevorzugt 0,0075 bis 0,75 Gew.-% und insbesondere 0,01 bis 0,5 Gew.-% mindestens eines Copolymers A der allgemeinen Formel (K2d)

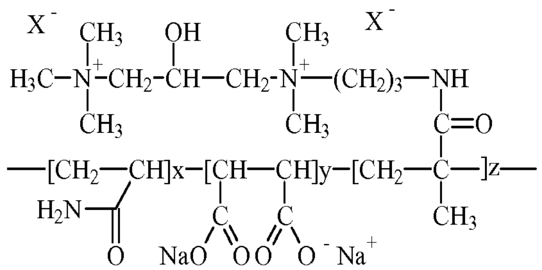

(K2d)

enthalten, in der gilt:

$$x + y + z \quad = \quad Q$$

Q steht für Werte von 3 bis 55000, vorzugsweise von 10 bis 25000, besonders bevorzugt von 50 bis 15000, weiter bevorzugt von 100 bis 10000, noch weiter bevorzugt von 500 bis 8000 und insbesondere von 1000 bis 5000,

x steht für (0 bis 0,5) Q, vorzugsweise für (0 bis 0,3) Q und insbesondere für die Werte 0, 1, 2, 3, 4, 5, wobei der Wert 0 bevorzugt ist,

y steht für (0,1 bis 0,95) Q, vorzugsweise für (0,5 bis 0,7) Q und insbesondere für Werte von 1 bis 24000, vorzugsweise von 5 bis 15000, besonders bevorzugt von 10 bis 10000 und insbesondere von 100 bis 4800,

z steht für (0,001 bis 0,5) Q, vorzugsweise für (0,1 bis 0,5) Q und insbesondere für Werte von 1 bis 12500, vorzugsweise von 2 bis 8000, besonders bevorzugt von 3 bis 4000 und insbesondere von 5 bis 2000.

Zusätzlich zu den vorstehend genannten Copolymeren oder an deren Stelle können die erfindungsgemäßen Mittel auch ein Copolymer enthalten, das aus Monomeren der Formel (K2a), Vinylsulfonsäure (bzw. deren Natriumsalz) und Acrylamid aufgebaut ist. Hierbei ist folgende Verteilung (in % der insgesamt im Polymer enthaltenen Monomere) bevorzugt:

| | |
|---|---|
| Monomer (K2a): | 0,1 bis 50 %, vorzugsweise 10 bis 50 % |
| Natriumvinylsulfonat: | 10 bis 95 %, vorzugsweise für 50 bis 70 % |
| Acrylamid: | 0 bis 50 %, vorzugsweise 0 bis 30 % |

Vorzugsweise enthält ein bevorzugtes Copolymer A folgende Anzahl an den jeweiligen Monomeren:

| | |
|---|---|
| Monomer (K2a): | Werte von 1 bis 12500, vorzugsweise von 2 bis 8000, besonders bevorzugt von 3 bis 4000 und insbesondere von 5 bis 2000 |
| Natriumvinylsulfonat: | Werte von 1 bis 24000, vorzugsweise von 5 bis 15000, besonders bevorzugt von 10 bis 10000 und insbesondere von 100 bis 4800 |
| Acrylamid: | Werte 0, 1, 2, 3, 4, 5, wobei der Wert 0 bevorzugt ist |

Zusammenfassend sind erfindungsgemäße Haarbehandlungsmittel bevorzugt, die - bezogen auf ihr Gewicht - 0,001 bis 5 Gew.-%, vorzugsweise 0,0025 bis 2,5 Gew.-%, besonders bevorzugt 0,005 bis 1 Gew.-%, weiter bevorzugt 0,0075 bis 0,75 Gew.-% und insbesondere 0,01 bis 0,5 Gew.-% mindestens eines Copolymers A der allgemeinen Formel

(K2e)

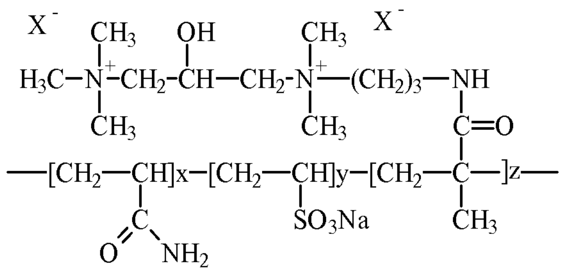

(K2e)

enthalten, in der gilt:

$$x + y + z = Q$$

| | |
|---|---|
| Q | steht für Werte von 3 bis 55000, vorzugsweise von 10 bis 25000, besonders bevorzugt von 50 bis 15000, weiter bevorzugt von 100 bis 10000, noch weiter bevorzugt von 500 bis 8000 und insbesondere von 1000 bis 5000, |
| x | steht für (0 bis 0,5) Q, vorzugsweise für (0 bis 0,3) Q und insbesondere für die Werte 0, 1, 2, 3, 4, 5, wobei der Wert 0 bevorzugt ist, |
| y | steht für (0,1 bis 0,95) Q, vorzugsweise für (0,5 bis 0,7) Q und insbesondere für Werte von 1 bis 24000, vorzugsweise von 5 bis 15000, besonders bevorzugt von 10 bis 10000 und insbesondere von 100 bis 4800, |
| z | steht für (0,001 bis 0,5) Q, vorzugsweise für (0,1 bis 0,5) Q und insbesondere für Werte von 1 bis 12500, vorzugsweise von 2 bis 8000, besonders bevorzugt von 3 bis 4000 und insbesondere von 5 bis 2000. |

Zusätzlich zu den vorstehend genannten Copolymeren oder an deren Stelle können die erfindungsgemäßen Mittel auch ein Copolymer enthalten, das aus Monomeren der Formel (K2a), Styrolsulfonsäure (bzw. deren Natriumsalz) und Acrylamid aufgebaut ist. Hierbei ist folgende Verteilung (in % der insgesamt im Polymer enthaltenen Monomere) bevorzugt:

| | |
|---|---|
| Monomer (K2a): | 0,1 bis 50 %, vorzugsweise 10 bis 50 % |
| Natriumstyrolsulfonat: | 10 bis 95 %, vorzugsweise für 50 bis 70 % |
| Acrylamid: | 0 bis 50 %, vorzugsweise 0 bis 30 % |

Vorzugsweise enthält ein bevorzugtes Copolymer A folgende Anzahl an den jeweiligen Monomeren:

| | |
|---|---|
| Monomer (K2a): | Werte von 1 bis 12500, vorzugsweise von 2 bis 8000, besonders bevorzugt von 3 bis 4000 und insbesondere von 5 bis 2000 |
| Natriumstyrolsulfonat: | Werte von 1 bis 24000, vorzugsweise von 5 bis 15000, besonders bevorzugt von 10 bis 10000 und insbesondere von 100 bis 4800 |
| Acrylamid: | Werte 0, 1, 2, 3, 4, 5, wobei der Wert 0 bevorzugt ist |

Zusammenfassend sind erfindungsgemäße Haarbehandlungsmittel bevorzugt, die - bezogen auf Ihr Gewicht - 0,001 bis 5 Gew.-%, vorzugsweise 0,0025 bis 2,5 Gew.-%, besonders bevorzugt 0,005 bis 1 Gew.-%, weiter bevorzugt 0,0075 bis 0,75 Gew.-% und insbesondere 0,01 bis 0,5 Gew.-% mindestens eines Copolymers A der allgemeinen Formel (K2f)

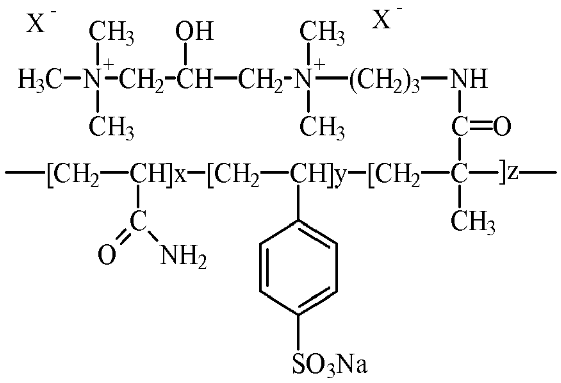

(K2f)

enthalten, in der gilt:

$$x + y + z = Q$$

- Q steht für Werte von 3 bis 55000, vorzugsweise von 10 bis 25000, besonders bevorzugt von 50 bis 15000, weiter bevorzugt von 100 bis 10000, noch weiter bevorzugt von 500 bis 8000 und insbesondere von 1000 bis 5000,
- x steht für (0 bis 0,5) Q, vorzugsweise für (0 bis 0,3) Q und insbesondere für die Werte 0, 1, 2, 3, 4, 5, wobei der Wert 0 bevorzugt ist,
- y steht für (0,1 bis 0,95) Q, vorzugsweise für (0,5 bis 0,7) Q und insbesondere für Werte von 1 bis 24000, vorzugsweise von 5 bis 15000, besonders bevorzugt von 10 bis 10000 und insbesondere von 100 bis 4800,
- z steht für (0,001 bis 0,5) Q, vorzugsweise für (0,1 bis 0,5) Q und insbesondere für Werte von 1 bis 12500, vorzugsweise von 2 bis 8000, besonders bevorzugt von 3 bis 4000 und insbesondere von 5 bis 2000.

Unabhängig davon, welche der bevorzugten Copolymere A der Formeln (K2c) bis (K2f) eingesetzt werden, sind erfindungsgemäße Haarbehandlungsmittel bevorzugt, die dadurch gekennzeichnet sind, daß das Verhältnis von (y : z) 4:1 bis 1:2, vorzugsweise 4:1 bis 1:1 beträgt.

Unabhängig davon, welche Copolymere A in den erfindungsgemäßen Mitteln eingesetzt werden, sind erfindungsgemäße Haarbehandlungsmittel bevorzugt, bei denen das Copolymer A eine Molmasse von 10000 bis 20 Millionen $gmol^{-1}$, vorzugsweise von 100000 bis 10 Millionen $gmol^{-1}$, weiter bevorzugt von 500000 bis 5 Millionen $gmol^{-1}$und insbesondere von 1,1 Millionen bis 2,2 Millionen $gmol^{-1}$ aufweist.

Ein höchst bevorzugtes Polymer, welches wie zuvor dargestellt aufgebaut ist, ist unter der Bezeichnung Polyquaternium-74 im Handel erhältlich.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare® CTH (Cosmetic Rheologies) und Synthalen® CR (3V Sigma) im Handel erhältlich.

Das Homopolymer wird bevorzugt in Form einer nichtwäßrigen Polymerdispersion eingesetzt. Solche Polymerdispersionen sind unter den Bezeichnungen Salcare® SC 95 und Salcare® SC 96 im Handel erhältlich.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel $G\text{-}O\text{-}B\text{-}N{+}R_aR_bR_c\ A^-$

G ist ein Anhydroglucoserest, beispielsweise Stärke- oder Celluloseanhydroglucose;

B ist eine divalente Verbindungsgruppe, beispielsweise Alkylen, Oxyalkylen, Polyoxyalkylen oder Hydroxyalkylen;

$R_a$, $R_b$ und $R_c$ sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in $R_a$, $R_b$ und $R_c$ vorzugsweise maximal 20 ist;

$A^-$ ist ein übliches Gegenanion und ist vorzugsweise Chlorid.

Kationische, also quaternisierte Cellulosen sind mit unterschiedlichem Substitutionsgrad, kationischer Ladungsdichte, Stickstoffgehalt und Molekulargewichten auf dem Markt erhältlich. Beispielsweise wird Polyquaternium-67 im Handel unter den Bezeichnungen Polymer® SL oder Polymer® SK (Amerchol) angeboten. Unter der Handelsbezeichnung Mirustyle® CP der Fa. Croda wird eine weitere höchst bevorzugte Cellulose angeboten. Diese ist eine Trimonium and Cocodimonium Hydroxyethylcellulose als derivatisierte Cellulose mit der INCI-Bezeichnung Polyquaternium-72. Polyquaternium-72 kann sowohl in fester Form als auch bereits in wässriger Lösung vorgelöst verwendet werden.

Weitere kationische Cellulosen sind unter den Bezeichnungen Polymer JR® 400 (Amerchol, INCI-Bezeichnung Polyquaternium-10) sowie Polymer Quatrisoft® LM-200 (Amerchol, INCI-Bezeichnung Polyquaternium-24). Weitere Handelsprodukte sind die Verbindungen Celquat® H 100 und Celquat® L 200. Schließlich liegt unter der Handelsbezeichnung Mirustyle® CP der Fa. Croda mit Trimonium and Cocodimonium Hydroxyethylcellulose eine weitere derivatisierte Cellulose mit der INCI-Bezeichnung Polyquaternium-72 vor. Polyquaternium-72 kann sowohl in fester Form als auch bereits in wässriger Lösung vorgelöst verwendet werden. Besonders bevorzugte kationische Cellulosen sind Polyquaternium-10, Polyquaternium-24, Polyquaternium-67 und Polyquaternium-72.

Geeignete kationische Guarderivate werden unter der Handelsbezeichnung Jaguar® vertrieben und haben die INCI-Bezeichnung Guar Hydroxypropyltrimonium Chloride. Weiterhin sind besonders geeignete kationische Guarderivate auch von der Fa. Hercules unter der Bezeichnung N-Hance® im Handel. Weitere kationische Guarderivate werden von der Fa. Cognis unter der Bezeichnung Cosmedia® vertrieben. Ein bevorzugtes kationisches Guarderivat ist das Handelsprodukt AquaCat® der Fa. Hercules. Bei diesem Rohstoff handelt es sich um ein bereits vorgelöstes kationisches Guarderivat. Die kationischen Guar-Derivate sind erfindungsgemäß bevorzugt.

Ein geeignetes Chitosan wird beispielsweise von der Firma Kyowa Oil& Fat, Japan, unter dem Handelsnamen Flonac® vertrieben. Ein bevorzugtes Chitosansalz ist Chitosoniumpyrrolidoncarboxylat, welches beispielsweise unter der Bezeichnung Kytamer® PC von der Firma Amerchol, USA, vertrieben wird. Weitere Chitosanderivate sind unter den Handelsbezeichnungen Hydagen® CMF, Hydagen® HCMF und Chitolam® NB/101 im Handel frei verfügbar.

Schließlich sind auch kationische Polymere auf der Basis von Zuckern erfindungsgemäß mit Vorzug verwendbar. Solche Zucker sind bevorzugt Alkyloligoglykoside, wie sie zuvor beschrieben wurden *(vide supra).* Die Quaternierung der Alkyloligoglukoside kann beispielsweise mit quarternären Ammoniumsalzen wie beispielsweise N,N-Dimethyl-N-(n-Alkyl)-N-(2-hydroxy-3-chloro-n-propyl)-ammoniumhalogen-iden durchgeführt werden. Die Kettenlänge der Alkylgruppe beträgt vorzugsweise 6 bis 30, bevorzugter 8 bis 24 Kohlenstoffatome.

Besonders bevorzugte Beispiele für die kationischen Alkyloligoglucoside sind die Verbindungen mit den INCI - Bezeichnungen Polyquaternium-77, Polyquaternium-78, Polyquaternium-79, Polyquaternium-80, Polyquaternium-81 und Polyquaternium-82. Höchst bevorzugt sind die kationischen Alkyloligoglucoside mit den Bezeichnungen Polyquaternium-77, Polyquaternium-81 und Polyquaternium-82.

Derartige Verbindungen können beispielsweise unter der Bezeichnung Poly Suga® Quat von der Fa. Colonial Chemical Inc. bezogen werden.

Die kationischen Alkyloligoglucoside werden in einer Gesamtmenge von 0,01 bis 10,0 Gew.%, vorzugsweise von 0,05 bis 5,0 Gew.%, noch bevorzugter von 0,1 bis 3,0 Gew.% und höchst bevorzugt in Mengen von 0,2 bis 2,0 Gew.% jeweils bezogen auf das Gesamtgewicht der Zusammensetzung verwendet. Erfindungsgemäß umfaßt ist selbstverständlich auch, daß Mischungen von kationischen Alkyloligoglucosiden verwendet werden können. Bevorzugt ist in diesem Falle, wenn jeweils ein langkettiges und ein kurzkettiges kationisches Alkyloligoglucosid gleichzeitig verwendet werden.

Ein weiteres kationisches Polymer kann auf der Grundlage von Ethanolamin erhalten werden. Das Polymer ist unter der Bezeichnung Polyquaternium-71 im Handel erhältlich.

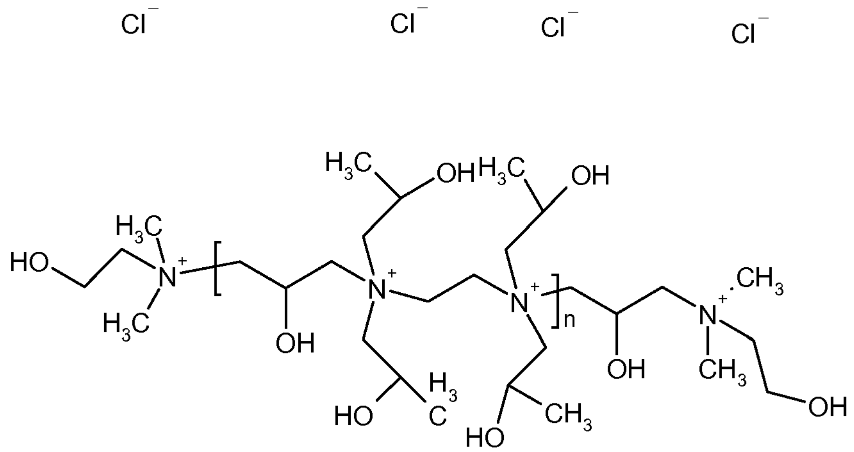

Dieses Polymer kann beispielsweise unter der Bezeichnung Cola® Moist 300 P von der Fa. Colonial Chemical Inc. bezogen werden.

Das Polyquaternium-71 wird in einer Gesamtmenge von 0,01 bis 10,0 Gew.%, vorzugsweise von 0,05 bis 5,0 Gew.%, noch bevorzugter von 0,1 bis 3,0 Gew.% und höchst bevorzugt in Mengen von 0,2 bis 2,0 Gew.% jeweils bezogen auf das Gesamtgewicht der Zusammensetzung verwendet. Weitere bevorzugte kationische Polymere sind

beispielsweise

- kationisierter Honig, beispielsweise das Handelsprodukt Honeyquat® 50,
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere mit der INCI - Bezeichnung Polyquaternium-7,
- Vinylpyrrolidon-Vinylimidazoliummethochlorid-Copolymere, wie sie unter den Bezeichnungen Luviquat® FC 370, FC 550 und der INCI-Bezeichnung Polyquaternium-16 sowie FC 905 und HM 552 angeboten werden,
- quaternisiertes Vinylpyrrolidon/Dimethylaminoethylmethacrylat, zum Beispiel Vinylpyrrolidon/Dimethylaminoethylmethacrylatmethosulfat Copolymer, das unter den Handelsbezeichnungen Gafquat® 755 N und Gafquat® 734 von der Firma Gaf Co., USA vertrieben wird und die INCI - Bezeichnung Polyquaternium-11,
- quaternierter Polyvinylalkohol,
- sowie die unter den Bezeichnungen Polyquaternium-2, Polyquaternium-17, Polyquaternium-18 und Polyquaternium-27 bekannten Polymeren mit quartären Stickstoffatomen in der Polymerhauptkette,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex® SF 40 angeboten werden.

Erfindungsgemäße amphotere Polymere sind solche Polymerisate, in denen sich eine kationische Gruppe ableitet von mindestens einem der folgenden Monomere:

(i) Monomeren mit quartären Ammoniumgruppen der allgemeinen Formel (Mono1 ),

$$R^1\text{-}CH{=}CR^2\text{-}CO\text{-}Z\text{-}(C_nH_{2n})\text{-}N^{(+)}R^2R^3R^4\ A^{(-)} \qquad \text{(Mono1)}$$

in der $R^1$ und $R^2$ unabhängig voneinander stehen für Wasserstoff oder eine Methylgruppe und $R^3$, $R^4$und $R^5$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und $A^{(-)}$ das Anion einer organischen oder anorganischen Säure ist,
(ii) Monomeren mit quartären Ammoniumgruppen der allgemeinen Formel (Mono2),

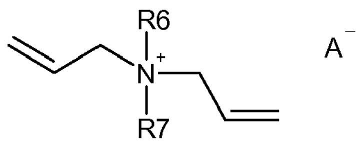

(Mono2)

worin $R^6$ und $R^7$ unabhängig voneinander stehen für eine ($C_1$ bis $C_4$)-Alkylgruppe, insbesondere für eine Methylgruppe und
$A^-$ das Anion einer organischen oder anorganischen Säure ist,
(iii) monomeren Carbonsäuren der allgemeinen Formel (Mono3),

$$R^8\text{-}CH{=}CR^9\text{-}COOH \qquad \text{(Mono3)}$$

in denen $R^8$ und $R^9$ unabhängig voneinander Wasserstoff oder Methylgruppen sind.

Besonders bevorzugt sind solche Polymerisate, bei denen Monomere des Typs (i) eingesetzt werden, bei denen $R^3$, $R^4$ und $R^5$ Methylgruppen sind, Z eine NH-Gruppe und $A^{(-)}$ ein Halogenid-, Methoxysulfat- oder Ethoxysulfat-Ion ist; Acrylamidopropyl-trimethyl-ammoniumchlorid ist ein besonders bevorzugtes Monomeres (i). Als Monomeres (ii) für die genannten Polymerisate wird bevorzugt Acrylsäure verwendet.

Besonders bevorzugte amphotere Polymere sind Copolymere, aus mindestens einem Monomer (Mono1) bzw. (Mono2) mit dem Momomer (Mono3), insbesondere Copolymere aus den Monomeren (Mono2) und (Mono3). Erfindungsgemäß ganz besonders bevorzugt verwendete amphotere Polymere sind Copolymerisate aus Diallyl-dimethylammoniumchlorid und Acrylsäure. Diese Copolymerisate werden unter der INCI-Bezeichnung Polyquaternium-22 unter anderem mit dem Handelsnamen Merquat® 280 (Nalco) vertrieben.

Darüber hinaus können die erfindungsgemäßen amphoteren Polymere neben einem Monomer (Mono1) oder (Mono2) und einem Monomer (Mono3) zusätzlich ein Monomer (Mono4) (iv) monomere Carbonsäureamide der allgemeinen Formel (Mono4),

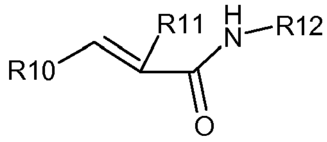

in denen $R^{10}$ und $R^{11}$ unabhängig voneinander Wasserstoff oder Methylgruppen sind und $R^{12}$ für ein Wasserstoffatom oder eine ($C_1$- bis $C_8$)-Alkylgruppe steht, enthalten.

Erfindungsgemäß ganz besonders bevorzugt verwendete amphotere Polymere auf Basis eines Comonomers (Mono4) sind Terpolymere aus Diallyldimethylammoniumchlorid, Acrylamid und Acrylsäure. Diese Copolymerisate werden unter der INCI-Bezeichnung Polyquaternium-39 unter anderem mit dem Handelsnamen Merquat® Plus 3330 (Nalco) vertrieben.

Die amphoteren Polymere können generell sowohl direkt als auch in Salzform, die durch Neutralisation der Polymerisate, beispielsweise mit einem Alkalihydroxid, erhalten wird, erfindungsgemäß eingesetzt werden.

Die zuvor genannten kationischen Polymere können einzeln oder in beliebigen Kombinationen miteinander verwendet werden, wobei Mengen zwischen 0,01 bis 10 Gew.%, bevorzugt, Mengen von 0,01 bis 7,5 Gew.% und ganz besonders bevorzugt in Mengen von 0,1 bis 5,0 Gew.% enthalten sind. Die allerbesten Ergebnisse werden dabei mit Mengen von 0,1 bis 3,0 Gew.% jeweils bezogen auf die Gesamtzusammensetzung des jeweiligen Mittels erhalten.

Im Rahmen dieser bevorzugten Ausführungsform, bei der das erfindungsgemäße Mittel als kosmetischen Wirkstoff mindestens eine erfindungsgemäß bevorzugte quaternäre Ammoniumverbindung als Komponente (b1) enthält, umfasst dieses bevorzugte Mittel zusätzlich zwingend als zweite Komponente (b2) mindestens einen Ölkörper. Zu den Ölkörpern zählt der Fachmann alle Stoffe, die sich bei 20°C bei pH 7 zu nicht mehr als 0,1 g in 100 g Wasser lösen und bei 1013 mbar einen Schmelzpunkt von unter 200°C haben.

Die Ölkörper sind in den erfindungsgemäßen Haarkonditioniermitteln bezogen auf ihr Gesamtgewicht bevorzugt in einer Menge von 0,01 bis 20 Gew.-%, besonders bevorzugt von 0,05 bis 15 Gew.% und insbesondere von 0,1 bis 10 Gew.% enthalten.

Die oben genannten Ölkörper sind von den Inhaltstoffen der Mischung der Komponente (b) des erfindungsgemäßen Mittels verschieden. Die Ölkörper werden bevorzugt ausgewählt aus mindestens einer Verbindung aus der Gruppe, die gebildet wird aus natürlichen Ölen, pflanzlichen Ölen, synthetischen Ölen, Fettsäuren, Fettalkoholen und, Wachsen.

Als natürliche (pflanzliche) Öle werden üblicherweise Triglyceride und Mischungen von Triglyceriden eingesetzt. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und Shea-Butter.

Weitere synthetische oder natürliche Öle sind beispielsweise

- Esteröle. Unter Esterölen sind zu verstehen die Ester von $C_6$ - $C_{30}$ - Fettsäuren mit $C_2$ - $C_{30}$ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, My ristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol® CC),

- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls® 90-018, Monomuls® 90-L12, Cetiol® HE oder Cutina® MD.

Als synthetische Öle kommen Silikonverbindungen in Betracht. Geeignete Silikone können ausgewählt sein unter:

(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:

a) substituierten oder unsubstituierten aminierten Gruppen;
b) (per)fluorierten Gruppen;
c) Thiolgruppen;
d) Carboxylatgruppen;
e) hydroxylierten Gruppen;
f) alkoxylierten Gruppen;
g) Acyloxyalkylgruppen;
h) amphoteren Gruppen;
i) Bisulfitgruppen;
j) Hydroxyacylaminogruppen;
k) Carboxygruppen;
l) Sulfonsäuregruppen; und
m) Sulfat- oder Thiosulfatgruppen;

(iii) linearen Polysiloxan(A)- Polyoxyalkylen(B)- Blockcopoylmeren vom Typ $(A-B)_n$ mit $n > 3$;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Als synthetische Ölkomponente kann weiterhin ein Dialkylether dienen.

Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Din-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.

Besonders bevorzugt ist der Di-n-octylether, der im Handel unter der Bezeichnung Cetiol® OE erhältlich ist.

Fettstoffe gehören ebenso zu den erfindungsgemäßen Ölkörpern. Unter Fettstoffen sind zu verstehen Fettsäuren, Fettalkohole sowie natürliche und synthetische Wachse, welche sowohl in fester Form als auch flüssig in wässriger Dispersion vorliegen können.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol®871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Als Fettalkohole können eingesetzt werden gesättigte, ein- oder mehrfach ungesättigte, verzweigte oder unverzweigte Fettalkohole mit $C_6$ - $C_{30}$-, bevorzugt $C_{10}$ - $C_{22}$- und ganz besonders bevorzugt $C_{12}$ - $C_{22}$- Kohlenstoffatomen.

Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden.

Als natürliche oder synthetische Wachse können eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Die erfindungsgemäßen Mittel aller Ausführungsformen weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die erfindungsgemäßen Mittel aller Ausführungsformen können weiterhin die Hilfs- und Zusatzstoffe enthalten, die üblicherweise herkömmlichen Stylingmitteln zugesetzt werden.

Als geeignete Hilfs- und Zusatzstoffe sind insbesondere zusätzliche Pflegestoffe zu nennen.

Besonders geeignete Pflegestoffe eignen sich aminofunktionelle Silikone, insbesondere die Silikone, die unter der INCI-Bezeichnung Amodimethicone zusammengefasst sind.

Die Mittel aller Ausführungsformen der Erfindung enthalten die aminofunktionellen Silikone bevorzugt in Mengen von 0,01 Gew.-% bis 15 Gew.-%, besonders bevorzugt von 0,05 bis 2 Gew.-%, bezogen auf das gesamte Mittel.

Als Pflegestoff einer anderen Verbindungsklasse kann das Mittel in allen vorgenannten Ausführungsformen beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff kann das erfindungsgemäße Mittel aller Ausführungsformen weiterhin mindestens ein Vitamin, ein Provitamin, eine Vitaminvorstufe und/oder eines derer Derivate enthalten.

Dabei sind erfindungsgemäß solche Vitamine, Provitamine und Vitaminvorstufen bevorzugt, die üblicherweise den Gruppen A, B, C, E, F und H zugeordnet werden. Ganz besonders bevorzugt wird als Pflegestoff D-Panthenol, gegebenenfalls in Kombination mit mindestens einem der oben genannten Silikonderivate eingesetzt.

Als Pflegestoff können die erfindungsgemäßen Mittel aller Ausführungsformen weiterhin mindestens einen Pflanzenextrakt enthalten.

Auch Mono- bzw. Oligosaccharide können als Pflegestoff in den erfindungsgemäßen Mitteln eingesetzt werden. Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Das Mittel kann weiterhin in allen seinen Ausführungsformen zusätzlich mindestens ein Lipid als Pflegestoff enthalten. Die erfindungsgemäßen Mittel enthalten die Lipide bevorzugt in Mengen von 0,01 bis 10 Gew.-%, insbesondere 0,1 bis 5 Gew.-%, bezogen auf die gesamte Anwendungszubereitung.

Durch Zugabe eines UV-Filters können sowohl die Mittel selbst, als auch die behandelten Fasern vor schädlichen Einflüssen von UV-Strahlung geschützt werden. Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In einer besonderen Ausführungsform enthält das erfindungsgemäße Mittel aller vorgenannten Ausführungsformen weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt. Die erfindungsgemäßen Mittel gemäß dieser Ausführungsform enthalten die direktziehenden Farbstoffe bevorzugt in einer Menge von 0,001 bis

20 Gew.-%, bezogen auf das gesamte Mittel.

Es ist erfindungsgemäß bevorzugt, dass die erfindungsgemäßen Mittel frei von Oxidationsfarbstoffvorprodukten sind. Oxidationsfarbstoffvorprodukte werden eingeteilt in sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Die Formulierung der erfindungsgemäßen Mittel aller vorgenannten Ausführungsformen kann in allen Kosmetika üblichen Formen erfolgen, beispielsweise in Form von Lösungen, die als Haarwasser oder Pump- oder Aerosolspray auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind.

Haarcremes und Haargele enthalten in der Regel Strukturanten und/oder verdickende Polymere, die dazu dienen, den Produkten die gewünschte Konsistenz zu verleihen. Strukturanten und/oder verdickende Polymere werden typischerweise in einer Menge von 0,1 bis 10 Gew.-%, bezogen auf das gesamte Produkt, eingesetzt. Mengen von 0,5 bis 5 Gew.-%, insbesondere 0,5 bis 3 Gew.-% sind bevorzugt.

Werden die erfindungsgemäßen Mittel als Aerosolschaum oder Aerosolspray konfektioniert, enthalten sie zusätzlich mindestens ein Treibmittel. Erfindungsgemäß geeignete Treibmittel sind beispielsweise ausgewählt aus $N_2O$, Dimethylether, $CO_2$, Luft, Alkanen mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und Mischungen daraus.

Gemäß einer bevorzugten Ausführungsform werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen.

Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.-% sind besonders bevorzugt. Aerosolsprays enthalten generell größere Mengen an Treibmittel. Bevorzugt wird das Treibmittel in diesem Fall in einer Menge von 30 bis 98 Gew.-%, bezogen auf das gesamte Produkt, eingesetzt. Mengen von 40 bis 95 Gew.-%, insbesondere von 50 bis 95 Gew.-% sind besonders bevorzugt.

Die Aerosolprodukte lassen sich in üblicher Art und Weise herstellen. In der Regel werden alle Bestandteile des jeweiligen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Folgende erfindungsgemäße kosmetische Mittel sind in einer Ausführungsform als Haarreinigungs-und/oder Haarkonditioniermittel besonders bevorzugt:

1. Haarreinigungs- und/oder Haarkonditioniermittel, enthaltend in einem kosmetischen Träger

    (a) 0,1 bis 60 Gew.-% mindestens eines Tensids aus der Gruppe, die gebildet wird aus anionischen, amphoteren/zwitterionischen, nichtionischen und/oder kationischen Tensiden, und

    (b) eine Mischung aus

        i) mindestens einem ($C_8$ bis $C_{20}$)-Alkan
        ii) mindestens einem bei 20°C und 1013 mbar flüssigen Polydimethylsiloxan und
        iii) mindestens einem Dimethiconol,

    wobei sich alle Mengenangaben auf das Gesamtgewicht des Haarreinigungs- und/oder Haarkonditioniermittels beziehen.

2. Haarreinigungs- und/oder Haarkonditioniermittel nach Punkt 1, dadurch gekennzeichnet, dass es - bezogen auf sein Gesamtgewicht -

    a) (i) 0,1 bis 10 Gew.-%, bevorzugt 0,2 bis 7,5 Gew.-% und insbesondere 0,3 bis 5 Gew.-% mindestens eines kationischen Tensids aus der Gruppe der quartären Ammoniumverbindungen, der Esterquats und der Amidoamine und/oder

(ii) 0,1 bis 20 Gew.-%, bevorzugt 0,5 bis 17,5 Gew.-% und insbesondere 1 bis 15 Gew.-% mindestens eines anionischen Tensids aus der Gruppe der Alkyl(ether)sulfate, der Ethercarbonsäuren, der Sulfobernsteinsäuremono- und -dialkylester, der Olefinsulfonate und der Acylsarcoside und/oder
(iii) 0,1 bis 15 Gew.-%, bevorzugt 0,5 bis 12,5 Gew.-% und insbesondere 1 bis 10 Gew.-% mindestens eines amphoteren/zwitterionischen Tensids aus der Gruppe der Alkylbetaine, der Alkylamidoalkylbetaine und der Alkylampho(di)acetate und/oder
(iv) 0,1 bis 15 Gew.-%, bevorzugt 0,5 bis 12,5 Gew.-% und insbesondere 1 bis 10 Gew.-% mindestens eines nichtionischen Tensids aus der Gruppe der Anlagerungsprodukte von Ethylenoxid und/oder Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, der Fettsäuremono- und -diester von Anlagerungsprodukten von Ethylenoxid an Glycerin, der Anlagerungsprodukte von Ethylenoxid an Rizinusöl und gehärtetes Rizinusöl, der Aminoxide, der Anlagerungsprodukte von Ethylenoxid an Fettsäurealkanolamide und Fettamine, und der Alkylpolyglucoside.

3. Haarreinigungs- und/oder Haarkonditioniermittel nach Punkt 2, dadurch gekennzeichnet, dass es mindestens ein Tensid aus den Gruppen

- (ii) und (iii) oder
- (ii) und (iii) und (i) oder
- (ii) und (iii) und (iv) oder
- (i) enthält.

4. Haarreinigungs- und/oder Haarkonditioniermittel nach einem der Punkte 1 bis 3, dadurch gekennzeichnet, dass es - bezogen auf sein Gesamtgewicht - zusätzlich 0,01 bis 10 Gew.- %, bevorzugt 0,05 bis 7,5 Gew.-% und insbesondere 0,1 bis 5 Gew.-% mindestens eines kationischen Polymeren enthält.
5. Haarreinigungs- und/oder Haarkonditioniermittel nach einem der Punkte 1 bis 4, dadurch gekennzeichnet, dass es zusätzlich 0,025 bis 7,5 Gew.-%, mehr bevorzugt 0,05 bis 5 Gew.-% und insbesondere 0,075 bis 3 Gew.-% mindestens eines Antischuppenwirkstoffs enthält.
6. Haarreinigungs- und/oder Haarkonditioniermittel nach einem der Punkte 1 bis 5, dadurch gekennzeichnet, dass es - bezogen auf sein Gesamtgewicht - zusätzlich 0,01 bis 20 Gew.- %, bevorzugt 0,05 bis 15 Gew.-% und insbesondere 0,1 bis 10 Gew.-% mindestens einer Öl-, Wachs- und/oder Fettkomponente enthält.
7. Haarreinigungs- und/oder Haarkonditioniermittel nach einem der Punkte 1 bis 6, dadurch gekennzeichnet, dass die besagten flüssigen Kohlenwasserstoffe ausgewählt werden aus verzweigten Kohlenwasserstoffen, insbesondere Isoparaffinen.
8. Haarreinigungs- und/oder Haarkonditioniermittel nach einem der Punkte 1 bis 7, dadurch gekennzeichnet, dass die ($C_8$ bis $C_{20}$)-Alkane ausgewählt werden aus mindestens einer Verbindung aus der Gruppe von Verbindungen mit der jeweiligen INCI-Bezeichnung C8-9 Isoparaffin, C8-10 Isoparaffin, C10-11 Isoparaffin, C10-13 Isoparaffin, C10-20 Isoparaffin, C11-12 Isoparaffin, C11-13 Isoparaffin, C11-15 Isoparaffin, C13-14 Isoparaffin, C13-16 Isoparaffin, Isodecane, Isoundecane, Isododecane, Isotetradecane, Isohexadecane.
9. Haarreinigungs- und/oder Haarkonditioniermittel nach einem der Punkte 1 bis 8, dadurch gekennzeichnet, dass die ($C_8$ bis $C_{20}$)-Alkane, insbesondere die ($C_1$ bis $C_6$)-Isoparaffine, in einer Menge von 25,0 bis 90,0 Gew.-%, insbesondere von 25,0 bis 50,0 Gew.-%, jeweils bezogen auf das Gesamtgewicht der Mischung (a), in der besagten Mischung (a) enthalten sind.
10. Haarreinigungs- und/oder Haarkonditioniermittel nach einem der Punkte 1 bis 9, dadurch gekennzeichnet, dass das bei 20°C und 1013 mbar flüssige Polydimethylsiloxan eine Viskosität von 1,5 bis 20000 cst, insbesondere von 1,5 bis 5000 cSt, jeweils gemessen bei 25 °C mit Hilfe eines Glaskapillarviskosimeters nach der Dow Corning Corporate Testmethode CTM 0004 vom 20. Juli 1970.
11. Haarreinigungs- und/oder Haarkonditioniermittel nach einem der Punkte 1 bis 10, dadurch gekennzeichnet, dass das Dimethiconol ausgewählt wird aus mindestens einer Verbindung der Formeln (I) und/oder (II)

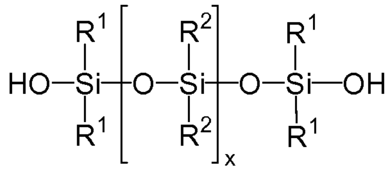 (I)

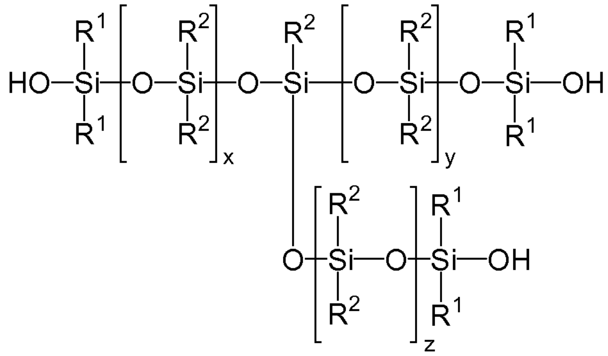 (II)

wobei in den Formeln (I) und (II)

- $R^1$ für Wasserstoff, einen Methylrest, einen $C_2$ bis $C_{30}$ linearen, gesättigten oder ungesättigten Kohlenwasserstoffrest, einen Phenylrest und/oder einen Arylrest,
- $R^2$ für Wasserstoff, einen Methylrest, einen $C_2$ bis $C_{30}$ linearen, gesättigten oder ungesättigten Kohlenwasserstoffrest, einen Phenylrest und/oder einen Arylrest, und
- x, y und z jeweils unabhängig voneinander für eine ganze Zahl von 0 bis 50.000 stehen.

12. Haarreinigungs- und/oder Haarkonditioniermittel nach einem der Punkte 1 bis 11, dadurch gekennzeichnet, dass das Dimethiconol eine Viskosität zwischen 1.000 und 5.000.000 cPs, bevorzugte zwischen 10.000 und 3.000.000 cPs, besonders bevorzugt zwischen 50.000 und 2.000.000 cPs, jeweils gemessen bei 25 °C mit Hilfe eines Glaskapillarviskosimeters nach der Dow Corning Corporate Testmethode CTM 0004 vom 20. Juli 1970.
13. Haarreinigungs- und/oder Haarkonditioniermittel nach einem der Punkte 1 bis 12, dadurch gekennzeichnet, dass das Dimethiconol in einer Menge von 20 bis 30 Gew.-%, insbesondere von 22 bis 28 Gew.-%, jeweils bezogen auf das Gesamtgewicht der Mischung (a) in der besagten Mischung (a) enthalten ist.
14. Haarreinigungs- und/oder Haarkonditioniermittel nach einem der Punkte 1 bis 13, dadurch gekennzeichnet, dass die Mischung (a) bezogen auf das Gesamtgewicht des Mittels in einer Menge von 0,01 bis 15,0 Gew.-%, insbesondere von 0,1 bis 10,0 Gew.-%, enthalten ist.

Folgende erfindungsgemäße kosmetische Mittel sind in einer Ausführungsform als Haarkonditioniermittel besonders bevorzugt:

1. Haarkonditioniermittel, enthaltend in einem kosmetischen Träger

(a1) mindestens eine quaternäre Ammoniumverbindung, und
(a2)mindestens einen Ölkörper, und
(b) eine Mischung aus

i) mindestens einem ($C_8$ bis $C_{20}$)-Alkan
ii) mindestens einem bei 20°C und 1013 mbar flüssigen Polydimethylsiloxan und
iii) mindestens einem Dimethiconol.

2. Haarkonditioniermittel nach Punkt 1, dadurch gekennzeichnet, dass die quaternäre Ammoniumverbindung ausgewählt wird aus mindestens einer Verbindung der Gruppe, die gebildet wird aus

a1-1) der Esterquats und/oder
a1-2) der quarternären Imidazoline der Formel (Tkat2),

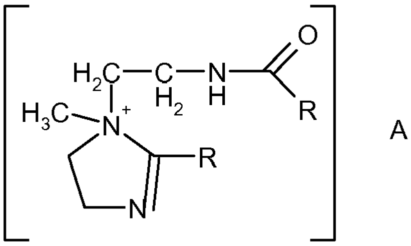

in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder

a1-3) der kationische Tenside der Formel (Tkat1-1)

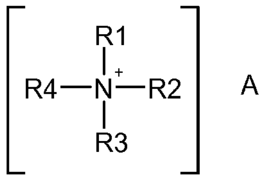

In welcher die Reste R1, R2, R3 und R4 jeweils unabhängig voneinander stehen für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann und A steht für ein physiologisch verträgliches Anion, und/oder

a1-4) der Amine und/oder kationisierten Amine und/oder

a1-5) Poly(methacryloyloxyethyltrimethylammoniumverbindungen) und/oder;

a1-6) quaternisierten Cellulose-Derivaten, insbesondere Polyquaternium 10, Polyquaternium-24, Polyquaternium-27, Polyquaternium-67, Polyquaternium-72, und/oder

a1-7) kationischen Alkylpolyglycoside und/oder

a1-8) kationisiertem Honig und/oder

a1-9) kationischen Guar-Derivaten und/oder

a1-10) Chitosan und/oder

a1-11) polymeren Dimethyldiallylammoniumsalzen und deren Copolymeren mit Estern und Amiden von Acrylsäure und Methacrylsäure, insbesondere Polyquaternium-7 und/oder

a1-12) Copolymeren des Vinylpyrrolidons mit quaternierten Derivaten des Dialkylaminoalkylacrylats und -methacrylats, insbesondere Polyquaternium-11 und/oder

a1-13) Vinylpyrrolidon-Vinylimidazoliummethochlorid-Copolymeren, insbesondere Polyquaternium-16 und/oder

a1-14) quaterniertem Polyvinylalkohol und/oder

a1-15) Polyquaternium-74,

a1-16) Polyquaternium-71,

a1-17) mindestens ein kationisches Keratinhydrolysat der Formel (K1)

$$R'\text{-}X\text{-}R'' \qquad (K1),$$

in der

- R' für einen geradkettigen oder verzweigten, gesättigten oder ungesättigten Kohlenwasserstoffrest mit 11 bis 24 Kohlenstoffatomen steht,
- R" ein Protein, ein Peptid oder ein Proteinhydrolysat bedeutet,
- X für $-N^+(R^{III}_2)R^{IV}-$ oder $-N(R^{III})R^{IV}-$ oder $-C(O)-N(R^V)R^{VI}-$ steht,
- $R^{III}$ $-(CH_2)_x-CH_3$ mit x = 0 - 22 bedeutet und
- $R^{IV}$ $-CH_2-CH(OH)-CH_2-$ oder $-(CH_2)_x-$ mit x = 0 - 22 bedeutet;
- $R^V$ und $R^{VI}$ unabhängig voneinander für -H oder $-(CH_2)_x-CH_3$ mit x = 0 - 22 stehen; mit der Maßgabe, daß R" für Keratin oder ein Keratinhydrolysat stehen, in einer Gesamtmenge von 0,1 bis 10 Gew.-%, und

3. Haarkonditioniermittel nach Anspruch 1 oder 2, dadurch gekennzeichnet, dass quaternären Ammoniumverbindungen in einer Gesamtmenge von 0,1 bis 10 Gew.-% bevorzugt 0,1 bis 8,0 Gew.-%, vorzugsweise 0,2 bis 8,0 Gew.-%, weiter bevorzugt 0,5 bis 8,0 Gew.-%, noch weiter bevorzugt 0,5 bis 6 Gew.-%, noch weiter bevorzugt 1,0 bis 6 Gew.-% und insbesondere 1,0 bis 5,0 Gew.-% enthalten sind.

4. Haarkonditioniermittel nach einem der Punkte 1 bis 3, dadurch gekennzeichnet, dass der Ölkörper ausgewählt wird aus mindestens einer Verbindung aus der Gruppe, die gebildet wird aus natürlichen Ölen, pflanzlichen Ölen, synthetischen Ölen, Fettsäuren, Fettalkoholen und, Wachsen.

5. Haarkonditioniermittel nach einem der Punkte 1 bis 4, dadurch gekennzeichnet, dass Ölkörper in einer Menge von 0,01 bis 20 Gew.-%, bevorzugt von 0,05 bis 15 Gew.%, besonders bevorzugt von 0,1 bis 10 Gew.%, enthalten sind

6. Haarkonditioniermittel nach einem der Punkte 1 bis 5, dadurch gekennzeichnet, dass die besagten flüssigen Kohlenwasserstoffe ausgewählt werden aus verzweigten Kohlenwasserstoffen, insbesondere Isoparaffinen.

7. Haarkonditioniermittel nach einem der Punkte 1 bis 6, dadurch gekennzeichnet, dass die ($C_8$ bis $C_{20}$)-Alkane ausgewählt werden aus mindestens einer Verbindung aus der Gruppe von Verbindungen mit der jeweiligen INCI-Bezeichnung C8-9 Isoparaffin, C8-10 Isoparaffin, C10-11 Isoparaffin, C10-13 Isoparaffin, C10-20 Isoparaffin, C11-12 Isoparaffin, C11-13 Isoparaffin, C11-15 Isoparaffin, C13-14 Isoparaffin, C13-16 Isoparaffin, Isodecane, Isoundecane, Isododecane, Isotetradecane, Isohexadecane.

8. Haarkonditioniermittel nach einem der Punkte 1 bis 7, dadurch gekennzeichnet, dass die ($C_8$ bis $C_{20}$)-Alkane, insbesondere die ($C_1$ bis $C_6$)-Isoparaffine, in einer Menge von 25,0 bis 90,0 Gew.-%, insbesondere von 25,0 bis 50,0 Gew.-%, jeweils bezogen auf das Gesamtgewicht der Mischung (c), in der besagten Mischung (c) enthalten sind.

9. Haarkonditioniermittel nach einem der Punkte 1 bis 8, dadurch gekennzeichnet, dass das bei 20°C und 1013 mbar flüssige Polydimethylsiloxan eine Viskosität von 1,5 bis 20000 cst, insbesondere von 1,5 bis 5000 cSt, jeweils gemessen bei 25 °C mit Hilfe eines Glaskapillarviskosimeters nach der Dow Corning Corporate Testmethode CTM 0004 vom 20. Juli 1970.

10. Haarkonditioniermittel nach einem der Punkte 1 bis 9, dadurch gekennzeichnet, dass das Dimethiconol ausgewählt wird aus mindestens einer Verbindung der Formeln (I) und/oder (II)

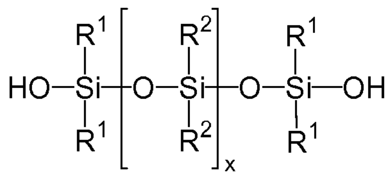

(I)

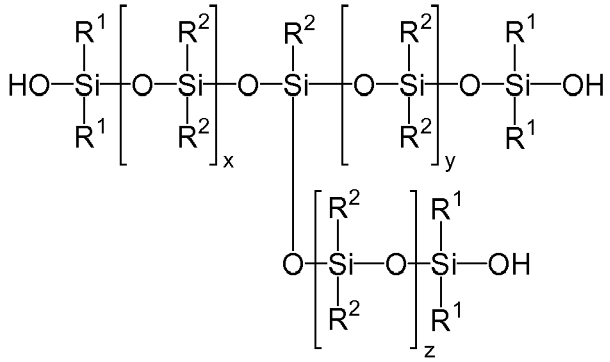

(II)

wobei in den Formeln (I) und (II)

- $R^1$ für Wasserstoff, einen Methylrest, einen $C_2$ bis $C_{30}$ linearen, gesättigten oder ungesättigten Kohlenwasserstoffrest, einen Phenylrest und/oder einen Arylrest,
- $R^2$ für Wasserstoff, einen Methylrest, einen $C_2$ bis $C_{30}$ linearen, gesättigten oder ungesättigten Kohlenwasserstoffrest, einen Phenylrest und/oder einen Arylrest, und
- x, y und z jeweils unabhängig voneinander für eine ganze Zahl von 0 bis 50.000 stehen.

11. Haarkonditioniermittel nach einem der Punkte 1 bis 10, dadurch gekennzeichnet, dass das Dimethiconol eine Viskosität zwischen 1.000 und 5.000.000 cPs, bevorzugte zwischen 10.000 und 3.000.000 cPs, besonders bevorzugt zwischen 50.000 und 2.000.000 cPs, jeweils gemessen bei 25 °C mit Hilfe eines Glaskapillarviskosimeters nach der Dow Corning Corporate Testmethode CTM 0004 vom 20. Juli 1970.

12. Haarkonditioniermittel nach einem der Punkte 1 bis 11, dadurch gekennzeichnet, dass das Dimethiconol in einer

Menge von 20 bis 30 Gew.-%, insbesondere von 22 bis 28 Gew.-%, jeweils bezogen auf das Gesamtgewicht der Mischung (c) in der besagten Mischung (c) enthalten ist.

13. Haarkonditioniermittel nach einem der Punkte 1 bis 12, dadurch gekennzeichnet, dass die Mischung (c) bezogen auf das Gesamtgewicht des Mittels in einer Menge von 0,01 bis 15,0 Gew.-%, insbesondere von 0,1 bis 10,0 Gew.-%, enthalten ist.

Ein zweiter Gegenstand der Erfindung ist ein Verfahren zur Behandlung keratinhaltiger Fasern, insbesondere menschlicher Haare, worin ein Mittel des ersten Erfindungsgegenstandes, auf die keratinhaltigen Fasern appliziert wird.

Die kosmetischen Mittel des zweiten erfindungsgemäßen Gegenstandes werden bevorzugt auf den Fasern belassen.

Ferner ist es erfindungsgemäß bevorzugt, dass die keratinhaltigen Fasern vor, nach oder während der Applikation des kosmetischen Mittels des zweiten Erfindungsgegenstandes in Form gebracht werden und diese Form durch das kosmetische Mittel des zweiten Erfindungsgegenstandes, fixiert wird.

Als erfindungsgemäß bevorzugt gelten für diesen zweiten Erfindungsgegenstand ebenso die zuvor genannten Ausführungsformen der Mittel der ersten Erfindungsgegenstandes *(vide supra).*

Ein dritter Gegenstand der Erfindung ist die Verwendung eines kosmetischen Mittels des ersten Erfindungsgegenstandes (insbesondere enthaltend mindestens einen festigenden Wirkstoff) zur temporären Umformung keratinhaltiger Fasern, insbesondere menschlicher Haare.

Ein vierter Gegenstand der Erfindung ist die kosmetische Verwendung eines Mittels des ersten Erfindungsgegenstandes, (insbesondere:

i) enthaltend die vorgenannten Komponenten (a1) und (a2); besonders bevorzugt nach einem Haarkonditioniermittel gemäß der Punkte 1 bis 14 des ersten Erfindungsgegenstandes, und/oder

ii) enthaltend als Komponente (a) zumindest 0,1 bis 60 Gew.-% mindestens eines Tensids aus der Gruppe, die gebildet wird aus anionischen, amphoteren/zwitterionischen, nichtionischen und/oder kationischen Tensiden

zur Verbesserung des Haargriffs und des Oberflächenglanzes von Haaren.

Als erfindungsgemäß bevorzugt gelten für diesen dritten, vierten und fünften Erfindungsgegenstand ebenso die zuvor genannten Ausführungsformen der Mittel der ersten Erfindungsgegenstandes *(vide supra).*

Beispiele

1. Stylingcreme

Folgende Stylingcreme wurde hergestellt:

| Rohstoff | [Gew.-%] |
|---|---|
| Glyzerin | 8,00 |
| Dow Corning CB 1502 Gum Organic Blend [1] | 1,00 |
| Edenor L2 SM [2] | 7,50 |
| Amaze [3] | 0,50 |
| Weichceresin® FL 400 [4] | 10,00 |
| Bienenwachs | 5,30 |
| Cosmedia Triple C [5] | 0,50 |
| Cetiol C 5 [6] | 0,50 |
| Luviskol K 90 [7] | 5,00 |
| Parfum | 0,30 |
| Neolone PE [8] | 0,60 |
| Milchsäure | 0,20 |

(fortgesetzt)

| Rohstoff | [Gew.-%] |
|---|---|
| Wasser | ad 100 |
| ' INCI-Bezeichnung: C11-13 Isoparaffin, Dimethiconol, Isohexadecane, Dimethicone (viskose Flüssigkeit mit einem Gehalt von 25 Gew.-% Dimethiconol) (DOW)<br>[2] Mischung von Palmitinsäure und Stearinsäure (INCI-Bezeichung Palmitic Acid, Stearic Acid) (BASF SE)<br>[3] INCI-Bezeichnung: Corn Starch Modified (pulverförmig) (Akzo Nobel)<br>[4] Vaseline-Vaselinöl-Wachs-Gemisch (INCI-Bezeichnung: Petrolatum) (Parafluid Mineralölgesellschaft)<br>[5] INCI-Bezeichnung: Polyquaternium-37, Dicaprylyl Carbonate, Lauryl Glucoside (BASF SE)<br>[6] INCI-Bezeichnung: Coco-Caprylate (BASF SE)<br>[7] Polyvinylpyrrolidon (ca. 20% Festkörper in Wasser; INCI-Bezeichnung: PVP) (BASF SE)<br>[8] 2-Methyl-2H-isothiazolin-3-one (ca. 1,55% in 2-Phenoxyethanol; INCI-Bezeichnung: Phenoxyethanol, Methylisothiazolinone) (DOW) | |

Die Stylingcreme wurde auf Haupthaar eines Probanden appliziert und das Haar zur Frisur gelegt. Es wurde ein feuchtebeständiges Styling und ein ausgewogener Konditioniereffekt erhalten. Die Stylingcreme war nicht klebrig und leicht und angenehm zu applizieren.

2. Haarreinigungsmittel

Es wurden die folgenden Haarshampoos A bis D hergestellt (die Mengenangaben beziehen sich - sofern nicht anders angegeben - auf Gew.-%):

| Rohstoffe | A | B | C | D |
|---|---|---|---|---|
| Texapon®[1] N70 | 15,0 | 15,0 | 12,0 | 18,0 |
| Dehyton ®[2] K | 10,0 | - | - | 15,0 |
| Dehyton®[3] G | - | 15,0 | 12,0 | - |
| Plantacare®[4] 818 UP | 4,0 | - | 2,0 | - |
| Dehyquart®[5] A CA | - | 0,5 | 0,1 | 0,2 |
| D-Panthenol | - | 0,2 | 0,2 | 0,1 |
| Nicotinsäureamid | 0,3 | - | 0,2 | 0,2 |
| Cutina®[6]HR | 0,4 | 0,6 | 0,5 | 0,3 |
| Cetiol®[7] HE | 1,0 | 0,8 | 0,4 | 0,6 |
| Polymer JR®[8] 400 | 0,3 | 0,5 | - | 0,3 |
| Polyquaternium-7 | - | - | - | 0,3 |
| Guar Hydroxypropyltrimonium Chloride | - | 0,1 | 0,2 | - |
| Dow Corning CB 1502®[9] | 0,5 | 0,7 | 1,0 | 1,2 |
| Aprikosenkernlöl | 0,1 | - | 0,2 | - |
| Citronensäure | 0,5 | 0,6 | 0,2 | 0,4 |
| Natrium Chlorid | 1,3 | 1,0 | 0,7 | 1,0 |

(fortgesetzt)

| Rohstoffe | A | B | C | D |
|---|---|---|---|---|
| Zink Pyrithion | - | 0,5 | 0,2 | 0,3 |
| Konservierungsmittel, Parfum | q.s. | q.s. | q.s. | q.s. |
| Wasser | ad 100 | ad 100 | ad 100 | ad 100 |

In den zuvor genannten Haarshampoos wurden die folgenden Handelsprodukte eingesetzt:

1 INCI-Bezeichnung: Sodium Laureth Sulfate; AS 68-73%; Cognis,
2 INCI-Bezeichnung: Cocamidopropyl Betaine; AS 29-32%; Cognis,
3 INCI-Bezeichnung: Disodium Cocoamphodiacetate; AS 30%; Cognis,
4 INCI-Bezeichnung: Coco Glucoside; AS 51-53%; Cognis,
5 INCI-Bezeichnung: Aqua, Cetrimonium Chloride, AS 24-26%; Cognis,
6 INCI-Bezeichnung: Hydrogenated Castor Oil; Cognis
7 INCI-Bezeichnung: PEG-7 Glyceryl Cocoate; Cognis
8 INCI-Bezeichnung: Polyquaternium-10; Dow
9 INCI-Bezeichnung: C11-13 Isoparaffin, Dimethiconol, Isohexadecane, Dimethicone (viskose Flüssigkeit mit einem Gehalt von 25 Gew.-% Dimethiconol) (DOW)

**Patentansprüche**

**1.** Kosmetisches Mittel zur kosmetischen Behandlung keratinhaltiger Fasern, insbesondere menschlicher Haare, enthaltend in einem kosmetisch akzeptablen Träger

(a) eine Mischung aus

i) mindestens einem ($C_8$ bis $C_{20}$)-Alkan
ii) mindestens einem bei 20°C und 1013 mbar flüssigen Polydimethylsiloxan und
iii) mindestens einem Dimethiconol, und

(b) mindestens einen haarkosmetischen Wirkstoff ausgewählt aus mindestens einem Wirkstoff der Gruppe, die gebildet wird aus festigenden Wirkstoffen, quaternären Ammoniumverbindungen und Tensiden.

**2.** Mittel nach Anspruch 2, **dadurch gekennzeichnet, dass** die besagten flüssigen Kohlenwasserstoffe ausgewählt werden aus verzweigten Kohlenwasserstoffen, insbesondere Isoparaffinen.

**3.** Mittel nach einem der Ansprüche 2 oder 3, **dadurch gekennzeichnet, dass** die ($C_8$ bis $C_{20}$)-Alkane ausgewählt werden aus mindestens einer Verbindung aus der Gruppe von Verbindungen mit der jeweiligen INCI-Bezeichnung C8-9 Isoparaffin, C8-10 Isoparaffin, C10-11 Isoparaffin, C10-13 Isoparaffin, C10-20 Isoparaffin, C11-12 Isoparaffin, C11-13 Isoparaffin, C11-15 Isoparaffin, C13-14 Isoparaffin, C13-16 Isoparaffin, Isodecane, Isoundecane, Isododecane, Isotetradecane, Isohexadecane.

**4.** Mittel nach einem der Ansprüche 1 bis 3, **dadurch gekennzeichnet, dass** die ($C_8$ bis $C_{20}$)-Alkane, insbesondere die ($C_1$ bis $C_6$)-Isoparaffine, in einer Menge von 25,0 bis 90,0 Gew.-%, insbesondere von 25,0 bis 50,0 Gew.-%, jeweils bezogen auf das Gesamtgewicht der Mischung (a), in der besagten Mischung (a) enthalten sind.

**5.** Mittel nach einem der Ansprüche 1 bis 4, **dadurch gekennzeichnet, dass** das bei 20°C und 1013 mbar flüssige Polydimethylsiloxan eine Viskosität von 1,5 bis 20000 cst, insbesondere von 1,5 bis 5000 cSt, jeweils gemessen bei 25 °C mit Hilfe eines Glaskapillarviskosimeters nach der Dow Corning Corporate Testmethode CTM 0004 vom 20. Juli 1970.

**6.** Mittel nach einem der Ansprüche 1 bis 5, **dadurch gekennzeichnet, dass** das Dimethiconol ausgewählt wird aus mindestens einer Verbindung der Formeln (I) und/oder (II)

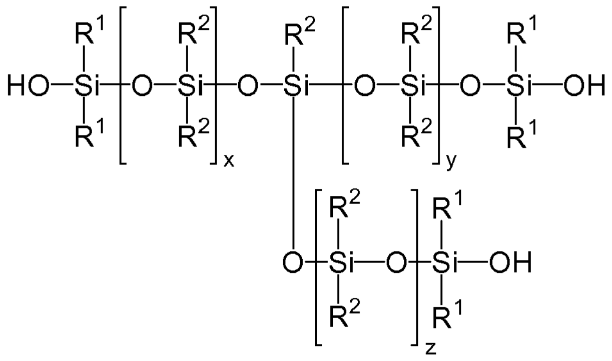

(II)

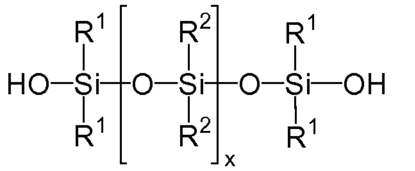

(I)

wobei in den Formeln (I) und (II)

- $R^1$ für Wasserstoff, einen Methylrest, einen $C_2$ bis $C_{30}$ linearen, gesättigten oder ungesättigten Kohlenwasserstoffrest, einen Phenylrest und/oder einen Arylrest,
- $R^2$ für Wasserstoff, einen Methylrest, einen $C_2$ bis $C_{30}$ linearen, gesättigten oder ungesättigten Kohlenwasserstoffrest, einen Phenylrest und/oder einen Arylrest, und
- x, y und z jeweils unabhängig voneinander für eine ganze Zahl von 0 bis 50.000 stehen.

7. Mittel nach einem der Ansprüche 1 bis 6, **dadurch gekennzeichnet, dass** das Dimethiconol eine Viskosität zwischen 1.000 und 5.000.000 cPs, bevorzugte zwischen 10.000 und 3.000.000 cPs, besonders bevorzugt zwischen 50.000 und 2.000.000 cPs, jeweils gemessen bei 25 °C mit Hilfe eines Glaskapillarviskosimeters nach der Dow Corning Corporate Testmethode CTM 0004 vom 20. Juli 1970.

8. Mittel nach einem der Ansprüche 1 bis 7, **dadurch gekennzeichnet, dass** das Dimethiconol in einer Menge von 20 bis 30 Gew.-%, insbesondere von 22 bis 28 Gew.-%, jeweils bezogen auf das Gesamtgewicht der Mischung (a) in der besagten Mischung (a) enthalten ist.

9. Mittel nach einem der Ansprüche 1 bis 8, **dadurch gekennzeichnet, dass** die Mischung (a) bezogen auf das Gesamtgewicht des Mittels in einer Menge von 0,01 bis 15,0 Gew.-%, insbesondere von 0,1 bis 10,0 Gew.-%, enthalten ist.

10. Mittel nach einem der Ansprüche 1 bis 9, **dadurch gekennzeichnet, dass** der festigende Wirkstoff ausgewählt wird aus mindestens einem festigenden Polymer und/oder mindestens einem Wachs.

11. Mittel nach einem der Ansprüche 1 bis 10, **dadurch gekennzeichnet, dass** der festigende Wirkstoff in einer Menge von 0,1 Gew.-% bis 25,0 Gew.-%, besonders bevorzugt von 1,0 Gew.-% bis 20,0 Gew.-%, ganz besonders bevorzugt von 1,0 Gew.-% bis 10,0 Gew.-%, jeweils bezogen auf das Gewicht des Mittels, enthalten ist.

12. Mittel nach einem der Ansprüche 1 bis 11, **dadurch gekennzeichnet, dass** es als einen kosmetischen Wirkstoff bezogen auf das Gesamtgewicht 0,1 bis 60 Gew.-% mindestens eines Tensids aus der Gruppe, die gebildet wird aus anionischen, amphoteren/zwitterionischen, nichtionischen und/oder kationischen Tensiden, enthält.

13. Mittel nach einem der Ansprüche 1 bis 12, **dadurch gekennzeichnet, dass** Haarreinigungs-und/oder Haarkonditioniermittel nach Anspruch 1, **dadurch gekennzeichnet, dass** es - bezogen auf sein Gesamtgewicht - als Komponente (a) enthält

(a) (i) 0,1 bis 10 Gew.-%, bevorzugt 0,2 bis 7,5 Gew.-% und insbesondere 0,3 bis 5 Gew.-% mindestens eines kationischen Tensids aus der Gruppe der quartären Ammoniumverbindungen, der Esterquats und der Amidoamine und/oder

(ii) 0,1 bis 20 Gew.-%, bevorzugt 0,5 bis 17,5 Gew.-% und insbesondere 1 bis 15 Gew.-% mindestens eines anionischen Tensids aus der Gruppe der Alkyl(ether)sulfate, der Ethercarbonsäuren, der Sulfobernsteinsäuremono- und -dialkylester, der Olefinsulfonate und der Acylsarcoside und/oder
(iii) 0,1 bis 15 Gew.-%, bevorzugt 0,5 bis 12,5 Gew.-% und insbesondere 1 bis 10 Gew.-% mindestens eines amphoteren/zwitterionischen Tensids aus der Gruppe der Alkylbetaine, der Alkylamidoalkylbetaine

und der Alkylampho(di)acetate und/oder
(iv) 0,1 bis 15 Gew.-%, bevorzugt 0,5 bis 12,5 Gew.-% und insbesondere 1 bis 10 Gew.-% mindestens eines nichtionischen Tensids aus der Gruppe der Anlagerungsprodukte von Ethylenoxid und/oder Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, der Fettsäuremono- und -diester von Anlagerungsprodukten von Ethylenoxid an Glycerin, der Anlagerungsprodukte von Ethylenoxid an Rizinusöl und gehärtetes Rizinusöl, der Aminoxide,
der Anlagerungsprodukte von Ethylenoxid an Fettsäurealkanolamide und Fettamine, und der Alkylpolyglucoside.

**14.** Mittel nach einem der Ansprüche 1 bis 13, **dadurch gekennzeichnet, dass** die quaternäre Ammoniumverbindung ausgewählt wird aus mindestens einer Verbindung der Gruppe, die gebildet wird aus

a1) der Esterquats und/oder
a2) der quarternären Imidazoline der Formel (Tkat2),

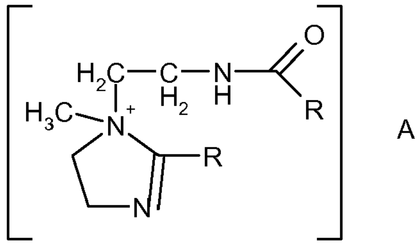

in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
a3) der kationische Tenside der Formel (Tkat1-1)

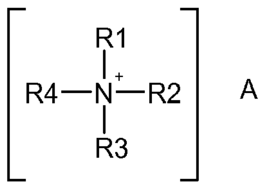

In welcher die Reste R1, R2, R3 und R4 jeweils unabhängig voneinander stehen für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann und A steht für ein physiologisch verträgliches Anion, und/oder
a4) der Amine und/oder kationisierten Amine und/oder
a5) Poly(methacryloyloxyethyltrimethylammoniumverbindungen) und/oder;
a6) quaternisierten Cellulose-Derivaten, insbesondere Polyquaternium 10, Polyquaternium-24, Polyquaternium-27, Polyquaternium-67, Polyquaternium-72, und/oder
a7) kationischen Alkylpolyglycoside und/oder
a8) kationisiertem Honig und/oder
a9) kationischen Guar-Derivaten und/oder
a10) Chitosan und/oder
a11) polymeren Dimethyldiallylammoniumsalzen und deren Copolymeren mit Estern und Amiden von Acrylsäure und Methacrylsäure, insbesondere Polyquaternium-7 und/oder
a12) Copolymeren des Vinylpyrrolidons mit quaternierten Derivaten des Dialkylaminoalkylacrylats und -methacrylats, insbesondere Polyquaternium-11 und/oder
a13) Vinylpyrrolidon-Vinylimidazoliummethochlorid-Copolymeren, insbesondere Polyquaternium-16 und/oder
a14) quaterniertem Polyvinylalkohol und/oder
a15) Polyquaternium-74,

a16) Polyquaternium-71,
a17) mindestens ein kationisches Keratinhydrolysat der Formel (K1) R'-X-R" (K1),
in der

- R' für einen geradkettigen oder verzweigten, gesättigten oder ungesättigten Kohlenwasserstoffrest mit 11 bis 24 Kohlenstoffatomen steht,
- R" ein Protein, ein Peptid oder ein Proteinhydrolysat bedeutet,
- X für $-N^+(R^{III}{}_2)R^{IV}-$ oder $-N(R^{III})R^{IV}-$ oder $-C(O)-N(R^V)R^{VI}-$ steht,
- $R^{III}$ $-(CH_2)_x-CH_3$ mit x = 0 - 22 bedeutet und
- $R^{IV}$ $-CH_2-CH(OH)-CH_2-$ oder $-(CH_2)_x-$ mit x = 0 - 22 bedeutet;
- $R^V$ und $R^{VI}$ unabhängig voneinander für -H oder $-(CH_2)_x-CH_3$ mit x = 0 - 22 stehen; mit der Maßgabe, daß R" für Keratin oder ein Keratinhydrolysat stehen, in einer Gesamtmenge von 0,1 bis 10 Gew.-%, und

**15.** Verfahren zur Behandlung keratinhaltiger Fasern, insbesondere menschlicher Haare, worin ein Mittel nach einem der Ansprüche 1 bis 14, auf die keratinhaltigen Fasern appliziert wird.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 1997038667 A
- DE 19757508